# EUROPEAN PATENT APPLICATION

(11) **EP 1 985 283 A1**
(43) Date of publication of application: **29.10.2008**
(21) Application number: 07706269.3
(22) Date of filing: 17.01.2007
(51) Int. Cl.: A61K 9/06, A61K 31/4162, A61K 45/00, A61K 47/34, A61L 15/44, A61P 19/00, A61P 19/02, A61P 35/00

(54) **GEL-FORMING COMPOSITION FOR MEDICAL USE, DEVICES FOR THE APPLICATION OF THE COMPOSITION, AND DRUG RELEASE CONTROLLING CARRIER**

(30) Priority: 18.01.2006 JP 2006009334
(71) Applicant: Next21 K.K., Tokyo 113-0033 (JP); The University of Tokyo, Bunkyo-ku, Tokyo 113-8654 (JP)
(72) Inventor: SASAKI, Nobuo, Tokyo 113-8654 (JP); TEI, Yuichi, Tokyo 113-8654 (JP); SUZUKI, Shigeki, Tokyo 113-0033 (JP); HAKUKAWA, Toru, Tokyo 113-0033 (JP)
(74) Representative: Moser & Götze
(86) International application number: PCT/JP2007/000017
(87) International publication number: WO 2007/083522

(57) **Abstract**

It is an object of the present invention to provide a gel-forming composition which permits on-site administration and can form a gel exhibiting prescribed strength and sustained drug release, devices for the administration thereof, and a kit for medical use. A gel-forming composition which is composed of the first composition comprising a drug, a first gelling agent represented by the general formula (I) and the like, and the first diluent and the second composition comprising the second gelling agent represented by the general formula (I) and the like and the second diluent; and application devices and kits for medical use, which can hold the gel-forming composition. X₁ - (OCH₂CH₂)ₙ - X₂ (I) wherein X₁ and X₂ are each -R¹COONHS (wherein R¹ is C₁₋₇ alkylene), -COR¹COONHS, -NOCOR¹-R² (wherein R² is maleimido), -R¹NH₂, -R¹SH, or -CO₂PhNO₂, and n is an integer of 80 to 1000.

## Description

### BACKGROUND OF THE INVENTION

### Field of the Invention

The present invention relates to a two-pack type gel-forming composition. In particular, the present invention relates to a gel-forming composition for mainly treating bone/cartilage related diseases such as osteoarthritis. It also relates to an administration device and a medical kit for administering the gel-forming composition.

### Description of the Related Art

Osteopathy mainly includes non-metabolic osteopathy such as bone fracture, bone deformity and spondylosis deformans, osteogenic sarcoma, myeloma, osteogenesis imperfecta, scoliosi, and metabolic osteopathy such as osteoporosis, osteomalacia, rickets, fibrous ostitis, renal osteodystrophy, Paget's disease of bone. For example, osteoporosis, a metabolic osteopathy, is a systemic disease characterized by the increase in bone fragility and easy bone fracture induced by low bone mass and the changes in microstructures of the osseous tissue. The major clinical symptoms thereof are hunchback and fracture of dorsolumbar bone, vertebral body, femoral neck, distal end of radius, rib, proximal end of humerus, etc. In the osseous tissue, osteogenesis and osteolysis by bone resorption occur repeatedly in a balanced way, where osteoblasts including osteoblasts precursor cells play a central role in osteogenesis, and osteoclasts play a central role in bone resorption. When the osteogenesis and osteolysis by bone resorption get out of balance, bone mass reduction is resulted. Therefore, there is a request to recover lost bone mass.

Articular diseases are characterized by the degeneration of articular cartilage (for example, chronic rheumatoid arthritis, osteoarthritis, or the like) as the major lesion. Cartilage is a tissue composed of collagen and proteoglycan, where release of proteoglycan from cartilage tissue is accelerated and decrease in proteoglycan synthesis in the tissue is initiated by a variety of causes. At the same time, release and activation of matrix metalloprotease such as collagenase-3 are stimulated, leading to the degradation of collagen in the cartilage tissue. These series of reactions promote hardening and destruction of the cartilage tissue, followed by the progression of the lesion leading to synovial hyperplasia, destruction of subchondral bone, and hypertrophy or bone neogenesis at the peripheral region of the joint, which are further followed by joint deformation, eventually resulting in dysfunction in serious cases. Articular diseases are most frequently observed in the knee joint but are also observed in the joints of elbow, hip, foot and finger. Among the articular diseases, osteoarthritis is the commonest disease suffered by the largest number of patients, while it is expected that the number of patients with the disease will increase in the aging society in the future, because aging is considered to be one of the etiologic factor of this disease.

Cartilage defect is a main etiologic factor for osteoarthritis. It is estimated that more than 70 percent of people over age 65 experience articular deformation. For treating osteoarthritis, antiphlogistic analgesic drugs and hyaluronic acid preparations are used. However, these drugs are used mainly to alleviate the pain that accompanies the cartilage degradation and hardening and destruction of subchondral bone, and are insufficient for providing desired effects. Stimulation of chondrogenesis, suppression of cartilage destruction, and induction and stimulation of differentiation of chondrocytes including chondrocyte precursor cells are expected to be effective in treating (or preventing) chondropathy. In the clinical field of osseous or articular diseases (e.g., chondropathy), excellent medical composition for preventing and treating the osseous or articular diseases are still desired.

Also, when partial loss of bone or cartilage tissue is caused by an accident, there is a demand to recover the lost part of bone or cartilage. Therefore, excellent medical compositions for recovering the lost bone tissue are desired. Various medicines for treating bone and cartilage related diseases are developed based on the request. However, medical compositions that are kept to be released for a prolonged period with appropriate concentration at the defect site of bone are not yet developed.

As a treatment for the bone and cartilage related diseases, regenerative medicine that is a therapeutic method of curing tissues lost utilizing artificially cultured cell is expected. However, this technique is physically taxing for patients because it is accompanied by cell transplantation. There is also a potential risk of contamination when cells are cultured. In consequence, in order to treat bone and cartilage related diseases such as osteoarthritis, medical composition, an administration device, and a medical kit for effectively performing induction of chondrogenesis are desired.

In such a background, it was found out that thieno indazoles derivative induces chondrogenesis (see Japanese Patent Laid-Open No. 2002-356419).

For an injury inflicted to skin or part of body tissue etc., wound coating materials covering such wound and recovering the lost body tissue are also expected. Also, for treatment of ulcers etc., wound coating materials covering the ulcer and administering a predetermined medicine for a prolonged period with appropriate concentration are expected.

The object of the present invention is to provide a gel-forming composition which can administer chondrogenesis prompter effectively, a medical composition having the gel-forming composition, an administration device, and a medical kit.

The object of the present invention is to provide an administration device, a medical kit, and a gel-forming composition which can induce osteogenesis or chondrogenesis without cell transplantation and have the advantage of being low invasive.

The object of the present invention is to provide a medical composition which is used for wound coating materials covering wound inflicted to skin and recovering partial loss of body tissue caused by an injury etc. It is also to provide an administration device and a medical kit for the medical composition.

The object of the present invention is to provide a drug release controlling carrier which can control drug release. In particular, the object of the present invention is to provide a drug release controlling carrier which can control drug release by applying pressure. Another object of the present invention is to provide a drug release controlling carrier which can maintain appropriate drug concentration by controlling drug release amount by repetitive pressure being applied to the carrier continuously. The present invention, among others, is made to provide a drug release controlling carrier having preferred drug release property under a condition of exerting continuous pressure on knee joints etc., a medical composition having the carrier, an administration device, and a medical kit.

### SUMMARY OF THE INVENTION

It is an object of the present invention to solve the above described problems by basically using a two-pack type gel-forming composition containing pharmaceutical agents such as osteogenesis/chondrogenesis promoter. And the present invention is based on the idea that since two components (two compositions) are mixed when a drug is administered to an affected area, a drug can be easily and simply administered to the affected area.

The present invention is also based on the idea that gel having the characteristics of appropriate rate of drug release, biocompatibility, resilience, and shape homeostasis can be obtained by using the mixture of predetermined gelators. In addition, the present invention is also based on the idea that the gelators are mixed immediately before the administration of a pharmaceutical agent since the gelation begins right after the mixture of the two components. Also, the present invention is based on the idea that a chondrogenesis promoter mixed with the gel-forming compositions can be administered on-site effectively by using medical device having two-pack type syringe that can accommodate two compositions separately.

A preferred embodiment of the present invention is based on an example that a drug release controlling carrier composed of polyrotaxane or pseudo-polyrotaxane has preferred drug release properties even when intermittent pressure is inflicted on the carrier.

The first aspect of the present invention relates to a gel-forming composition comprising a first composition and a second composition. The first composition and the second composition becoming gel state when mixed with each other, wherein the first composition comprises: a pharmaceutical agent; a first gelator comprising one or more than one kind of compound represented by the below-described general formula (I) or (II), or a compound including 3 to 8 repeating units represented by the below-described general formula (III); and a first diluent, and wherein the second composition comprises: a second gelator comprising one or more than one kind of compound represented by the below-described general formula (I) or (II), or a compound including 3 to 8 repeating units represented by the below-described general formula (III); and a second diluent.

X₁ - (OCH₂CH₂)ₙ - X₂ (I)

wherein X₁ and X₂ are the same or different, and each represents - R¹COONHS (where R¹ is a C₁₋₇ alkylene group), -COR¹COONHS, -NOCOR¹-R² (where R² is a maleimide group), -R¹NH₂, -R¹SH, or -CO₂PhNO₂ (where Ph is an o-, m-, or p-phenylene group), and n is an integer of 80 to 1000.

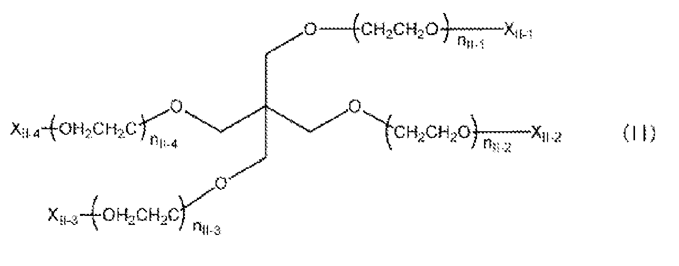

wherein X_{II-1} to X_{II-4} are the same or different and each represents - R¹COONHS (where R¹ is a C₁₋₇ alkylene group), -COR¹COONHS, -NOCOR¹-R² (where R² is a maleimide group), -R¹NH₂, -R¹SH, or -CO₂PhNO₂ (where Ph is an o-, m-, or p-phenylene group), and n_{II-1} to n_{II-4} are the same or different and each represents an integer of 20 to 250.

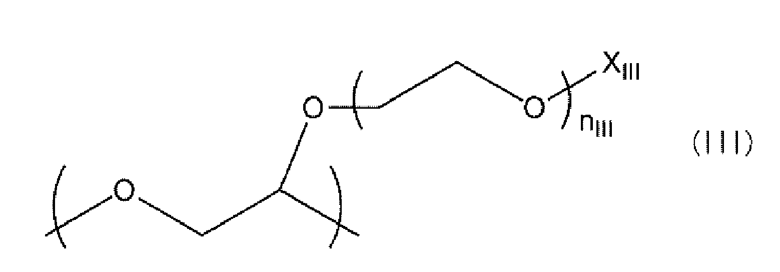

wherein X_{III} represents -R¹COONHS (where R¹ is a C₁₋₇ alkylene group), - COR¹COONHS, -NOCOR¹-R² (where R² is a maleimide group), -R¹NH₂, -R¹SH, or - CO₂PhNO₂ (where Ph is an o-, m-, or p-phenylene group), and n_{III} represents an integer of 10 to 150.

A preferred embodiment of the first aspect of the present invention relates to the above described gel-forming composition, wherein the first gelator comprises one or more than one kind of compound represented by the general formula (I) or (II), wherein X₁, X₂ or X_{II-1} to X_{II-4} are the same or different and each represents - NOCOR¹-R² or -R¹NH₂, and wherein the second gelator comprises one or more than one kind of compound represented by the general formula (I) or (II), wherein X₁, X₂ or X_{II-1} to X_{II-4} are the same or different and each represents -COR¹COONHS, -R¹SH, or -CO₂PhNO₂ (where Ph is an o-, m-, or p-phenylene group).

A preferred embodiment of the first aspect of the present invention relates to one of the above described gel-forming compositions, wherein the first gelator comprises one or more than one kind of compound represented by the general formula (I) or (II), wherein X₁, X₂ or X_{II-1} to X_{II-4} are the same or different and each represents -NOCOR¹-R² or -R¹NH₂, and number average molecular weight is 3x10³ to 4x10⁴, and wherein the second gelator comprises one or more than one kind of compound represented by the general formula (I) or (II), wherein X₁, X₂ or X_{II-1} to X_{II-4} are the same or different and each represents -COR¹COONHS, -R¹SH, or - CO₂PhNO₂ (where Ph is an o-, m-, or p-phenylene group), and number average molecular weight is 3 x 10³ to 4 x 10⁴.

A preferred embodiment of the first aspect of the present invention relates to one of the above described gel-forming compositions, wherein the diluent is one kind or a mixture of more than one kind of water, phosphate buffer solution, citrate buffer solution, phosphate buffered saline solution or physiologic saline solution.

A preferred embodiment of the first aspect of the present invention relates to one of the above described gel-forming compositions, wherein one or both of the first composition and the second composition further comprises stabilizing agent.

A preferred embodiment of the first aspect of the present invention relates to one of the above described gel-forming compositions, wherein the pharmaceutical agent is an osteogenesis/chondrogenesis promoter, a joint disease therapeutic agent, a preventive and/or therapeutic agent for bone/cartilage disease, a bone-regenerating agent, a bone resorption-suppressing substance, an angiogenesis promoter, or an anticancer agent.

A preferred embodiment of the first aspect of the present invention relates to one of the above described gel-forming compositions, wherein the pharmaceutical agent comprises thieno indazole derivative represented by below-described general formula (IV), prostaglandin A1 derivative, vitamin D derivative, vitamin K₂ derivative, eicosapentaenoic acid, benzyl sulfonic acid, bisphosphonic acid derivative, sex hormone derivative, phenol sulfo phthalein derivative, benzothiopyran or benzothiepine derivative, menatetrenone derivative, helioxanthine derivative, dihydrobenz [c] thiophene compound, tricyclic thiophene compound, Indian hedgehog (Ihh) or agonist of the signaling pathway thereof, PTH-related peptide or agonist of the signaling pathway thereof, pharmaceutically acceptable salt thereof, or pharmaceutically acceptable solvate thereof:

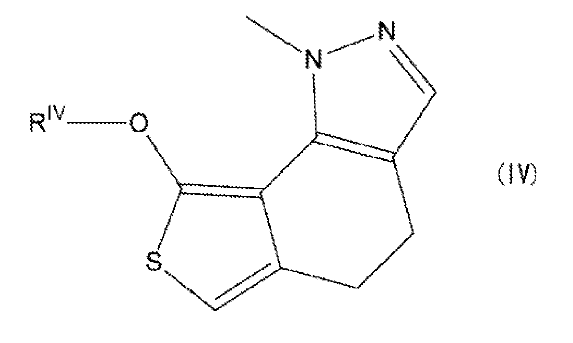

wherein R^{1V} represents a carboxyamido group.

A preferred embodiment of the first aspect of the present invention relates to one of the above described gel-forming compositions, wherein the pharmaceutical agent comprises trafermin, pharmaceutically acceptable salt thereof, pharmaceutically acceptable solvate thereof or prodrug thereof.

A preferred embodiment of the first aspect of the present invention relates to one of the above described gel-forming compositions, wherein the pharmaceutical agent comprises a gene.

A preferred embodiment of the first aspect of the present invention relates to one of the above described gel-forming compositions, further comprising controlled-release formulation for controlling release of the pharmaceutical agent after gel formation is completed.

A preferred embodiment of the first aspect of the present invention relates to one of the above described gel-forming compositions, wherein the pharmaceutical agent is a ligand.

A preferred embodiment of the first aspect of the present invention relates to one of the above described gel-forming compositions, used as a wound coating material.

A preferred embodiment of the first aspect of the present invention relates to one of the above described gel-forming compositions, used as a wound coating material, wherein the pharmaceutical agent comprises trafermin, a pharmaceutically acceptable salt thereof, a pharmaceutically acceptable solvate thereof or a prodrug thereof.

A preferred embodiment of the first aspect of the present invention relates to one of the above described gel-forming compositions, further comprising polyrotaxane or pseudo-polyrotaxane as a drug release controlling carrier.

A preferred embodiment of the first aspect of the present invention relates to one of the above described gel-forming compositions, further comprising polyrotaxane or pseudo-polyrotaxane as a drug release controlling carrier, wherein the polyrotaxane or the pseudo-polyrotaxane comprises: one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and one or more than one kind of chain molecules selected from a group consisting of "polyalkylene glycol, copolymer of polyalkylene glycol, polyether, polyolefin, polyorganosiloxane, derivative thereof, pharmaceutically acceptable salts thereof, and pharmaceutically acceptable solvate thereof".

A preferred embodiment of the first aspect of the present invention relates to one of the above described gel-forming compositions, further comprising pseudo-polyrotaxane as drug release controlling carrier, wherein the pseudo-polyrotaxane comprises: cyclodextrin; and polyethylene glycol, polypropylene glycol, or copolymer of polyethylene glycol and polypropylene glycol.

A preferred embodiment of the first aspect of the present invention relates to one of the above described gel-forming compositions. The gel-forming composition is used for medical treatment of bone/cartilage disease. And the gel-forming composition further comprises polyrotaxane or pseudo-polyrotaxane as drug release controlling carrier, wherein the polyrotaxane or the pseudo-polyrotaxane comprises: one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and one or more than one kind of chain molecules selected from a group consisting of "polyalkylene glycol, copolymer of polyalkylene glycol, polyether, polyolefin, polyorganosiloxane, derivative thereof, pharmaceutically acceptable salt thereof, and pharmaceutically acceptable solvate thereof".

A preferred embodiment of the first aspect of the present invention relates to one of the above described gel-forming compositions. The gel-forming composition is used for medical treatment of knee joint disease. And the gel-forming composition further comprises polyrotaxane or pseudo-polyrotaxane as drug release controlling carrier, wherein the polyrotaxane or the pseudo-polyrotaxane comprises: one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and one or more than one kind of chain molecules selected from a group consisting of "polyalkylene glycol, copolymer of polyalkylene glycol, polyether, polyolefin, polyorganosiloxane, derivative thereof, pharmaceutically acceptable salt thereof, and pharmaceutically acceptable solvate thereof".

A preferred embodiment of the first aspect of the present invention relates to one of the above described gel-forming compositions. The gel-forming composition is used for medical treatment of knee joint disease. And the gel-forming composition further comprises pseudo-polyrotaxane as drug release controlling carrier, wherein the pseudo-polyrotaxane comprises: cyclodextrin; and polyethylene glycol, polypropylene glycol, or copolymer of polyethylene glycol and polypropylene glycol.

A preferred embodiment of the first aspect of the present invention relates to one of the above described gel-forming compositions. The gel-forming composition is used for medical treatment of knee joint disease. The gel-forming composition further comprises pseudo-polyrotaxane as drug release controlling carrier, wherein the pseudo-polyrotaxane comprises: cyclodextrin; and polyethylene glycol, polypropylene glycol, or copolymer of polyethylene glycol and polypropylene glycol, and wherein the pharmaceutical agent comprises thieno indazoles derivative represented by the above-described general formula (IV), prostaglandin A1 derivative, vitamin D derivative, vitamin K₂ derivative, eicosapentaenoic acid, benzyl sulfonic acid, bisphosphonic acid derivative, sex hormone derivative, phenol sulfo phthalein derivative, benzothiopyran or benzo thiepine derivative, menatetrenone derivative, helioxanthine derivative, dihydrobenz [c] thiophene compound, or tricyclic thiophene compound, Indian hedgehog (Ihh) or agonist of the signaling pathway thereof, PTHrP (PTH-related peptide) or agonist of the signaling pathway thereof, pharmaceutically acceptable salt thereof, or pharmaceutically acceptable solvate thereof.

The second aspect of the present invention relates to an administration device (1) containing one of the above mentioned gel-forming compositions. The administration device (1) comprises: two containing rooms (2, 3) containing the first composition and the second composition, the two containing rooms (2, 3) spatially isolating the compositions from each other; moving parts (4a, 4b) being connected to the two containing rooms (2, 3), the moving parts (4a, 4b), wherein the first composition and the second composition can move within the moving parts (4a, 4b) respectively; a mixing part (5) mixing the first composition and the second composition, the first composition and the second composition having moved through the moving parts (4a, 4b); and a discharge part (6) discharging the first composition and the second composition, the first composition and the second composition having been mixed in the mixing part (5).

A preferred embodiment of the second aspect of the present invention relates to the above described administration device, wherein the discharge part (6) has a spray port.

A preferred embodiment of the second aspect of the present invention relates to one of the above described administration device comprising: two containing rooms (2, 3) containing the first composition and the second composition, the two containing rooms (2, 3) spatially isolating the compositions from each other; moving parts (4a, 4b) being connected to the two containing rooms (2, 3), the moving parts (4a, 4b), wherein the first composition and the second composition can move within the moving parts (4a, 4b) respectively; discharge parts (6a, 6b) discharging the first composition and the second composition separately, the first composition and the second composition having moved through the moving parts (4a, 4b).

A preferred embodiment of the second aspect of the present invention relates to the above described administration device, wherein the discharge parts (6a, 6b) have spray ports.

The third aspect of the present invention relates to a medical kit comprising: a pharmaceutical agent; a first gelator comprising one or more than one kind of a compound represented by the general formula (I) or (II), or a compound including 3 to 8 repeating units represented by the general formula (III); a second gelator comprising one or more than one kind of a compound represented by the general formula (I) or (II), or a compound including 3 to 8 repeating units represented by the general formula (III); a diluent; and an administration device comprising a two pack type syringe, wherein the administration device comprises: two containing rooms (2, 3) containing a first composition and a second composition, the first composition including the pharmaceutical agent, the first gelator, and the diluent, the second composition including the second gelator and the diluent, the two containing rooms (2, 3) spatially isolating the compositions from each other; moving parts (4a, 4b) being connected to the two containing rooms (2, 3), the moving parts (4a, 4b), wherein the first composition and the second composition can move within the moving parts (4a, 4b) respectively; a mixing part (5) mixing the first composition and the second composition, the first composition and the second composition having moved through the moving parts (4a, 4b); and a discharge part (6) discharging the first composition and the second composition, the first composition and the second composition having been mixed in the mixing part (5), or wherein the administration device comprises: two containing rooms (2, 3) containing the first composition and the second composition, the two containing rooms (2, 3) spatially isolating the compositions from each other; moving parts (4a, 4b) being connected to the two containing rooms (2, 3), the moving parts (4a, 4b), wherein the first composition and the second composition can move within the moving parts (4a, 4b) respectively; and discharge parts (6a, 6b) discharging the first composition and the second composition separately, the first composition and the second composition having moved through the moving parts (4a, 4b).

A preferred embodiment of the third aspect of the present invention relates to the above described medical kit, wherein the discharge part/parts (6 or 6a, 6b) have spray port/ports.

A preferred embodiment of the third aspect of the present invention relates to one of the above described medical kit, further comprising stabilizing agent.

The fourth aspect of the present invention relates to a drug release controlling carrier for controlling drug release comprising polyrotaxane or pseudo-polyrotaxane, wherein the polyrotaxane or the pseudo-polyrotaxane comprises: one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivatives thereof, pharmaceutically acceptable salt thereof, and pharmaceutically acceptable solvates thereof", and one or more than one kind of chain molecules selected from a group consisting of "polyalkylene glycol, copolymer of polyalkylene glycol, polyether, polyolefin, polyorganosiloxane, derivative thereof, pharmaceutically acceptable salts thereof, and pharmaceutically acceptable solvate thereof".

A preferred embodiment of the fourth aspect of the present invention relates to the above described drug release controlling carrier for controlling drug release comprising pseudo-polyrotaxane, wherein the pseudo-polyrotaxane comprises: cyclodextrin as the cyclic molecule; and polyethylene glycol, polypropylene glycol, or copolymer of polyethylene glycol and polypropylene glycol as the chain molecule.

A preferred embodiment of the fourth aspect of the present invention relates to one of the above described drug release controlling carrier for controlling drug release by applying pressure. The drug release controlling carrier comprises pseudo-polyrotaxane, wherein the pseudo-polyrotaxane comprises: cyclodextrin as the cyclic molecule; and polyethylene glycol, polypropylene glycol, or copolymer of polyethylene glycol and polypropylene glycol as the chain molecule.

A preferred embodiment of the fourth aspect of the present invention relates to one of the above described drug release controlling carrier used for treatment of knee joint. The drug release controlling carrier comprises pseudo-polyrotaxane, wherein the pseudo-polyrotaxane comprises: cyclodextrin as the cyclic molecule; and polyethylene glycol, polypropylene glycol, or copolymer of polyethylene glycol and polypropylene glycol as the chain molecule.

The present invention can provide a gel-forming composition that can administer pharmaceutical agents such as osteogenesis/chondrogenesis promoters effectively, a medical composition comprising the gel-forming composition, and an administration device and a medical kit for the gel-forming composition because the gel-forming composition of the present invention has desirable strength and sustained drug release, and can be administered to an affected area on-site.

The present invention can provide an administration device, a medical kit, and a gel-forming composition which are low invasive to patients since the present invention can induce osteogenesis and chondrogenesis without being accompanied by cell transplantation.

As demonstrated in the example described below, the gel formed from the gel-forming composition of the present invention has preferred resilience and morphological stability in vivo even when it is administrated, for example, in a defective part of knee joint cartilage which is filled with joint fluid. The gel also has strong adhesiveness with the osteocyte surface membrane. Therefore the present invention can provide a gel-forming composition which can be effectively used for treating bone/cartilage disease, and a medical kit containing the gel-forming composition. In addition, the present invention can provide a gel-forming composition which can generate gel having the characteristics of preferred resilience because the resilience of the gel can be adjusted by adjusting conditions such as the concentration of gelator, the kinds of gelator, the kinds of diluent, etc., as needed.

As demonstrated in the example described below, the gel obtained by the present invention has characteristics of excellent sustained drug release which enables gradual drug release with appropriate concentration and adequate drug release rate, and maintains constant release amount of drug for prolonged time. Therefore, the present invention can provide a gel-forming composition and a medical kit containing the gel-forming composition which are particularly effective for bone/cartilage diseases.

The kit of the present invention can administer gel containing a pharmaceutical agent directly to a drug administration site such as a cartilage defect site. The kit of the present invention eliminates the need for oral administration of a pharmaceutical agent or transplantation of regeneration cells. And since the kit of the present invention can administer a pharmaceutical agent directly to an affected area ("on-site" administration) by using arthroscope etc., a low invasive treatment can be provided. Namely, the kit of the present invention, together with the gel-forming composition of the present invention, acts as an excellent drug delivery system (hereinafter referred to as DSS).

The composition of the present invention also acts as a wound coating material for covering wound inflicted to skin and recovering partial loss of body tissue caused by an injury. Covering a wound area by the gel also prevent germs from penetrating into the wound. A composition containing a predetermined pharmaceutical agent further promotes recovery of a wound.

As demonstrated in the example described below, a drug release controlling carrier composed of polyrotaxane or pseudo-polyrotaxane has preferred drug release properties even when intermittent pressure is inflicted on the carrier. Therefore, the present invention can provide a drug release controlling carrier having a preferred drug release property even under a condition of continuous pressure, and a medical component having the carrier, an administration device, and a medical kit.

### BRIEF DESCRIPTION OF THE DRAWINGS

Fig. 1 is a schematic diagram showing an administration device according to an embodiment of the present invention.
Fig. 2 is a schematic diagram showing an administration device according to a certain embodiment of the present invention.
Fig. 3 shows photographs, in place of a diagram, showing temporal changes of a gel in PBS. Fig. 3 (A) shows the gel one week later. Fig.3 (B) shows the gel fourteen weeks later. Fig. 3 (C) shows the gel twenty eight weeks later.
Fig. 4 shows photographs, in place of a diagram, showing the results of toluidine blue staining for examining sustained release of the chondrogenesis promoting factor TM in the gel of the present invention. Fig. 4(A) shows the results of toluidine blue staining using TM concentrations (0 µM and 1 µM), 7 days (7d) or 14 days (14d) had elapsed after the gelation is completed. Fig. 4(B) shows the results of toluidine blue staining using TM concentrations (0.1 µM and 10 µM) one week has elapsed after the gelation is completed.
Fig. 5 shows photographs, in place of a diagram, showing a two-component mixing syringe (the admonition device of the present invention). Fig. 5(A) shows the overall view. Fig. 5(B) shows the vicinity of the moving part. Fig. 5(C) shows the vicinity of the discharge part.
Fig. 6 shows photographs, in place of a diagram, showing an administration device having two spray ports at the discharge parts. Fig. 6(A) shows the overall view. Fig. 6(B) shows the vicinity of the moving parts. Fig. 6(C) shows the vicinity of the discharge parts.
Fig. 7 shows photographs, in place of a diagram, illustrating pig's knee cartilage defect model. Fig. 7(A) shows a defect site before injecting a gel-forming composition. Fig. 7(B) shows a gel filled in the defect site. Fig. 7(C) shows the cartilage which was taken out of PBS and wiped out 5 minutes after the gel-forming composition was injected.
Fig. 8 is a photograph, in place of a diagram, for examining the effect of chondrogenesis promotion.
Fig. 9 is a graph showing an influence of iteration pressure on releasing capacity of the drug release controlling carrier of the present invention when gelator solvent No. 1 was used.
Fig. 10 is a graph showing release amounts of placebo from gels formed from the gelator solvent No. 1 in accordance the presence or absence of pressure on the gels. Fig. 11 is a graph showing an influence of iteration pressure on releasing capacity of the drug release controlling carrier of the present invention when gelator solvent No. 2 was used.
Fig. 12 is a graph showing release amounts of placebo from gels formed from the gelator solvent No. 2 in accordance with the presence or absence of pressure on the gels.

### DESCRIPTION OF THE PREFFERED EMBODIMENTS

### Gel-forming Composition of the Present Invention

A gel-forming composition comprises a first composition and a second composition. The first composition and the second composition become gel state when mixed with each other. The first composition comprises: a pharmaceutical agent; a first gelator comprising one or more than one kind of compound represented by below-described general formula (I) or (II), or a compound including 3 to 8 repeating units represented by below-described general formula (III); and a first diluent. The second composition comprises: a second gelator comprising one or more than one kind of compound represented by below-described general formula (I) or (II), or a compound including 3 to 8 repeating units represented by below-described general formula (III); and a second diluent. The second composition may comprise the same or different pharmaceutical agent as the first composition.

X₁ - (OCH₂CH₂)ₙ - X₂ (I)

(wherein X₁ and X₂ are the same or different and each represents - R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n is an integer of 80 to 1000.)

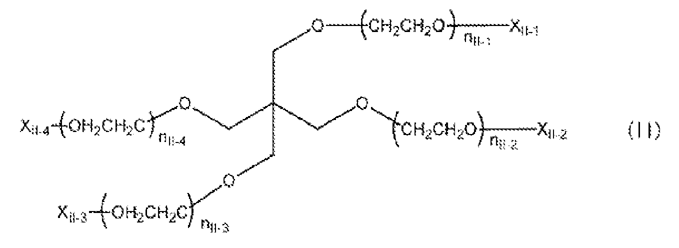

(wherein X_{II-1} to X_{II-4} are the same or different and each represents - R¹COONHS (where R¹ is a C₁₋₇ alkylene group), -COR¹COONHS (where R¹ is a C₁₋₇ alkylene group), -NOCOR¹-R² (where R¹ is a C₁₋₇ alkylene group, and R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{II-1} to n_{II-4} are the same or different and each represents an integer of 20 to 250.)

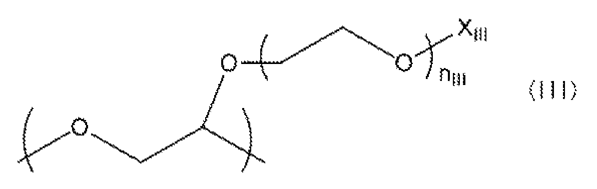

(wherein X_{III} represents -R¹COONHS (where R¹ is a C₁₋₇ alkylene group), - COR¹COONHS, -NOCOR¹-R² (where R¹ is a C₁₋₇ alkylene group, R² is a maleimide group), -R¹NH₂ (where R¹ is a C₁₋₇ alkylene group), -R¹SH (where R¹ is a C₁₋₇ alkylene group), or -CO₂PhNO₂ (where Ph is an o-, m-, or p-phenylene group), and n_{III} represents an integer of 10 to 150.)

In the general formula (I), X₁ and X₂ are the same or different and each represents -R¹COONHS, -COR¹COONHS, -NOCOR¹-R² (where R² is a maleimide group), -R¹NH₂, -R¹SH, or -CO₂PhNO₂, and n is an integer of 80 to 1000. In the general formula (I), X₁ and X₂ preferably are the same. R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. And, n is an integer of 80 to 1000, and preferably is 100 to 500.

In the general formula (II), X_{II-1} to X_{II-4} are the same or different and each represents -R¹COONHS, -COR¹COONHS, -NOCOR¹-R² (where R² is a maleimide group), -R¹NH₂, -R¹SH, or -CO₂PhNO₂, and n_{II-1} to n_{II-4} are the same or different and each represents an integer of 20 to 250. In the general formula (II), X_{II-1} to X_{II-4} preferably are the same. R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. And n_{II-1} to n_{II-4} preferably are the same and each represents an integer of 20 to 250 and preferably is an integer of 40 to 200.

In the general formula (III), X_{III} represents -R¹COONHS, -COR¹COONHS, - NOCOR¹-R² (where R² is a maleimide group), -R¹NH₂, -R¹SH, or -CO₂PhNO₂, and n_{III} represents an integer of 10 to 150. In the general formula (III), R¹ is a C₁₋₇ alkylene group, preferably is a C₁₋₅ alkylene group, and more preferably is a C₁₋₂ alkylene group or a C₅ alkylene group. Ph is an o-, m-, or p-phenylene group, and preferably is a p-phenylene group. The number of repeating units of the compound represented by the general formula (III) preferably is 3 to 5, more preferably is 4. And the ends of the compound represented by the general formula (III) preferably have functional groups represented by X_{III} of the general formula (III). In particular, it is preferred that the both ends of the compound have a group represented by a formula X_{III}-(OCH₂CH₂) _{nIII}-O-CH₂-CH (-O- (CH₂CH₂O) _{nIII}-X_{III}) -CH₂- and a group represented by a formula X_{III}-(OCH₂CH₂) _{nIII}-O-CH₂-CH (-O- (CH₂CH₂O) _{nIII}-X_{III}) -CH₂-O-.

The preferred embodiment of the gel-forming composition of the present invention is the above described gel-forming composition, wherein the first gelator comprises one or more than one kind of compound represented by the general formula (I) or (II), where X₁, X₂ or X_{II-1} to X_{II-4} are the same or different and each represents -NOCOR¹-R² or -R¹NH₂, and wherein the second gelator comprises one or more than one kind of compound represented by the general formula (I) or (II), where X₁, X₂ or X_{II-1} to X_{II-4} are the same or different and each represents - COR¹COONHS, -R¹SH, or -CO₂PhNO₂ (where Ph is an o-, m-, or p-phenylene group).

The first gelator mixed in the first composition containing a pharmaceutical agent, as demonstrated in the example below, preferably is a compound having a functional group of -NOCOR¹-R² or -R¹NH₂ (in particular, a compound having a functional group of -R¹NH₂). It is also preferred that the second gelator, mixed with the first gelator, undergoes rapid crosslinkage reaction and the resultant gel has predetermined strength (gel resilience) and morphological stability. It is also preferred that the gel, which is obtained by mixing the first gelator with the second gelator, has a certain steric structure and so keep releasing proper amount of a pharmaceutical agent contained therein. From this perspective, it is preferred that the second gelator mixed in the second composition is a compound having a functional group of - COR¹COONHS, -R¹SH, or - CO₂PhNO₂ (where Ph is an o-, m-, or p-phenylene group) (in particular, a compound having a functional group of -COR¹COONHS). Also, as demonstrated in the example below, both the first and the second gelators preferably are compounds represented by the general formula (II). In particular, as demonstrated in the example below, a combination of a propyl amine group and a succinimidyl group is preferred as a combination of the functional group of the first gelator and that of the second gelator.

Another preferred embodiment of the gel-forming composition of the present invention comprises the first gelator and the second gelator, wherein the first gelator comprises one or more than one kind of compound represented by the general formula (I) or (II), where X₁, X₂ or X_{II-1} to X_{II-4} are the same or different and each represents -NOCOR¹-R² (where R¹ is a C₁₋₅ alkylene group) or -R¹NH₂, (where R¹ is a C₁₋₅ alkylene group), and wherein the second gelator comprises one or more than one kind of compound represented by the general formula (I) or (II), where X₁, X₂ or X_{II-1} to X_{II-4} are the same or different and each represents -COR¹COONHS, -R¹SH (where R¹ is a C₁₋₅ alkylene group), or -CO₂PhNO₂ (where Ph is a p-phenylene group).

The molecular weight of the compound composing the gelator is not specifically limited, but, as demonstrated in the example below, a preferred gel can be obtained from a compound (gelator) of molecular weight (number average molecular weight) 3 x 10³ to 4 x 10⁴, and a more preferred gel can be obtained from a compound of molecular weight 1 x 10⁴ to 3 x 10⁴.

The concentration of the gelator is not specifically limited if the concentration of the compound composing the gelator in the first composition or the second composition are in the range of 5 mM to 20 mM. As demonstrated in the example below, a particularly preferred gel can be obtained from the gelator with the above concentration. In particular, it is assumed that a particularly preferred gel can be obtained when the concentration of the component in each composition is from 6 mM to 30 mM.

The compound represented by the general formula (I) or (II), and the compound including 3 to 8 repeating units represented by the general formula (III) are commercially available and can be synthesized by a known method.

The compound represented by the general formula (I) or (II), and the compound including 3 to 8 repeating units represented by the general formula (III) may respectively be a pharmaceutically acceptable salt thereof or a solvate thereof.

The term "pharmaceutically acceptable salt" represents salts of the above described compounds, particularly represents pharmaceutically acceptable salts of the above described compounds. The term "pharmaceutically acceptable" in this specification means that something is not deleterious to the recipient thereof. The polyphosphoric acid of the present invention can be made to salt by a known method. The examples of the "pharmaceutically acceptable salt" includes: the alkaline metal salts such as sodium salt, potassium salt, and lithium salt; the alkaline earth metal salts such as calcium salt, and magnesium salt; the metal salts such as aluminum salt, iron salt, zinc salt, copper salt, nickel salt, and cobalt salt; the inorganic salts such as ammonium salt; and the organic amine salts such as t-octyl amine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N-dibenzylethylenediamine salt, chloroprocaine salt, procaine salt, diethanolamine salt, N-benzil-N-phenethylamine salt, piperazine salt, tetramethylammonium salt, tris (hydroxymethyl) aminomethane salt. Of these salts, as polyphosphoric acid salt, alkaline metal salt is preferred, and sodium salt is more preferred. In this specification, "the pharmaceutically acceptable salt" may include not only anhydrous salt but also hydrate salt. These salts, for example, are ionized in vivo, and act the same as the above described compounds.

The term "pharmaceutically acceptable solvate" represents solvates of the above described compounds. The solvate herein includes a hydrate. The agent of the present invention may absorb moisture, be attached with absorption water, and be hydrated when it is left in the atmosphere or recrystallized. The solvates thus obtained are also included in the "pharmaceutically acceptable solvate". These solvates are ionized in vivo, and act the same as the above described compounds.

A preferred embodiment of the gel-forming composition of the present invention comprises publicly known pharmaceutically acceptable diluent as a diluent. Specific examples of the diluent are solvents including one kind or a mixture of more than one kind of water, phosphate buffer solution, citrate buffer solution, phosphate buffered saline or physiologic saline solution.

The acid level of the diluent (or solvent) is not specifically limited, but it includes pH 3 to pH 11. As demonstrated in Example 4 below, in order to improve the strength of the gel obtained to some extent, the acid level is preferably from pH 5 to pH 10, more preferably from pH 6 to pH 9, and further preferably from pH 7 to pH 8. The molar concentration (M) of the diluent is not specifically limited, but it includes 1 mM to 1 M. As demonstrated in Example 5 below, in order to improve the strength of the gel obtained to some extent, the molar concentration is preferably from 5 mM to 300 mM, more preferably from 10mM to 200mM, and further preferably from 15 mM to 100 mM.

A preferred embodiment of the gel-forming composition of the present invention is the above described gel-forming composition wherein one or both of the first composition and the second composition include stabilizing agents. As the stabilizing agent, a publicly known stabilizing agent used for a polymer etc., in particular, a pharmaceutically acceptable stabilizing agent can be used as needed. The gel obtained from the gel-forming composition of the present invention maintains its strength for a prolonged period mainly in vivo. Since enzymes such as protease exists in vivo, the gel may be dissolved by the enzymes. So a preferred embodiment of the gel-forming composition of the present invention, for example, includes inhibitors such as protease inhibitor. As the inhibitors, publicly known enzyme inhibitors can be used as needed. Specific examples of the protease inhibitor include one or more than one kind of 4-(2-aminoethyl) benzene sulfonyl fluoride, aprotinin, bestain, calpains inhibitor I, calpains inhibitor II, chymostain, 3,4-dichloroisocoumain, E-64, EDTA, EGTA, Lactacystin, Leupeptin, MG-115, MG-132, pepstain A, phenylmethyl sulfonyl fluoride, proteasome inhibitor I, p-toluene sulfonyl-L-lycine chloromethyl ketone, p-toluene sulfonyl-L-phenylalanine chloromethyl ketone, or tylosine inhibitor. These protease inhibitors are commercially available, and the inhibition concentrations thereof are publicly known. A preferred embodiment of the gel formed from the gel-forming composition of the present invention maintains the gel strength for a prolonged period and has sustained drug release. Therefore, the gel-forming composition of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dosage). In general, the gel-forming composition (per 1 mL) contains 0.1 µg to 0.5 mg of protease inhibitor. The amount included may be 1 µg to 0.1 mg, or may be 10 µg to 0.1 mg. By adjusting the amount of stabilizing agent added, degradability of the gel can be controlled, thereby adjusting release property of pharmaceutical agents. Namely, the present invention can provide a method for controlling the drug release by adjusting the amount of stabilizing agent added to the gel composition.

A preferred embodiment of the gel-forming composition of the present invention is the above described gel-forming composition wherein the pharmaceutical agent comprises an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, or an anticancer agent. A preferred embodiment of the gel-forming composition of the present invention is the above described gel-forming composition wherein the pharmaceutical agent comprises thieno indazole derivative represented by the below described general formula (IV). The thieno indazole derivative represented by the general formula (IV) can be produced, for example, by a method disclosed in the Japanese Patent Laid-Open No. 2002-356419. It is desirable that effective dose of the pharmaceutical agent that provides a predetermined efficacy is contained in the gel-forming composition of the present invention.

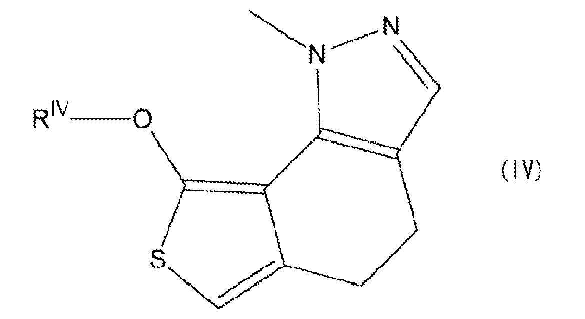

wherein R^{1V} represents a carboxyamido group (-CONH₂).

### Osteogenesis/Chondrogenesis Promoter

As the osteogenesis/chondrogenesis promoter, a publicly known agent for inducing osteogenesis or chondrogenesis can be used as needed. In particular, a chondrogenesis promoter is, for example, a 2- [1- (2, 2-diethoxy-ethyl) -3- (3-p-trill-uredido) -2, 3-dihydro-1H-indole-3-iru] -N-p-trill-acetamide (2-[1-(2,2- Diethoxy-ethyl)-3- (3-p-tolyl-ureido) -2, 3-dihydro-1H-indol-3-yl] -N-p-tolyl-acetamide) which is disclosed in WO 02/087620. As a chondrogenesis promoter, for example, osteogenesis promoting factor can be used. The osteogenesis promoting factor is generally referred to as BMP (bone morphogenetic protein). The BMP is a substance for bone/cartilage induction which acts on undifferentiated mesenchymal cells from outside, differentiating them to chondrocyte or osteoblasts. As the osteogenesis promoting factor, for example, BMP1 to 13 can be used. The BMP used as a pharmaceutical agent of the present invention may be either one of the agent obtained by genetic recombination or the purified agent taken form Dunn osteogenic sarcoma (see Takaoka, K., Biomedical Research, 2 (5) 466-471 (1981)).

### Joint Disease Treating Agent

Examples of the joint disease treating agent include: anti-inflammatory steroid agents such as p38MAP kinase inhibitor (thiazole-based compound etc., disclosed in WO00/64894), matrix metalloprotease inhibitor (MMPI), prednisolone, hydrocortisone, methylpredinisolone, dexabethamethasone, and bethamethasone; and non-steroid anti-inflammatory analgesic agents such as indometacin, diclofenac, loxoprofen, ibuprofen, piroxicam, and sulindac.

### Bone/Cartilage Disease Preventing or Treating Agent

Examples of the bone/cartilage disease preventing or treating agent include one or mixtures of more than one kind of the following substances: non-peptide osteogenesis-promoting substances such as prostaglandin A1 derivative, vitamin D derivative, vitamin K₂ derivative, eicosapentaenoic acid derivative, benzylphosphonic acid, bisphosphonic acid derivative, sex hormone derivative, phenolsulfophthalein derivative, benzothiopyran or benzothiepine derivative, thienoindazole derivative, menatetrenone derivative, helioxanthine derivative; and a hardly soluble peptide osteogenesis-promoting substance. These substances can be obtained by a known method. A bone/cartilage disease preventing agent includes one or both of a cartilage disease preventive agent and an agent preventing the situation that a bone/cartilage disease develops. A bone/cartilage disease, also referred to as a bone/cartilage related disease in this specification, includes one or both of a disease associated with bone and a disease associated with cartilage. Examples of bone/cartilage diseases include coxarthrosis, defect or change in shape of a bone/cartilage by an external injury, osteochondrodysplasia, dysostosis, achondroplasia, hypochondroplasia, chondroectodermal dysplasia, spondyloepiphyseal dysplasia congenita, metaphyseal chondrodysplasia, spondylometaphyseal dysplasia, and osteoporosis.
The present invention is intended particularly to stimulate cartilage reproduction. So the drug release controlling carrier and the medical compositions containing the carrier of the present invention can be advantageously used for treatment of defect or change in shape of a bone/cartilage by an external injury. Coxarthrosis, to which the present invention is applied, includes unexplained primary coxarthrosis and secondary coxarthrosis. And examples of causes of the secondary coxarthrosis include dysplastic hip, (congenital) hip dislocation, Perthes disease, external injury (transcervical fracture, hip dislocation, etc.), inflammation, infection.

### Bone-Regenerating Agent

Examples of the bone-regenerating agent include one kind or a mixture of more than one kind of the following substances: calmodulin; actinomycin D; cyclosporin A; glucosamine sulfate; glucosamine hydrochloride; marrow extract; calcium phosphate; lactic acid/ glycolic acid/ ε-caprolactone copolymer; platelet rich plasma; or human marrow mesenchymal cell. These substances can be obtained by a known method.

### Bone Resorption-Suppressing Substance

Examples of the bone resorption-suppressing substance include one kind or a mixture of more than one kind of estrogenic agent, calcitonin, and bisphosphonate. These substances can be obtained by a known method.

### Angiogenesis Promoter

Examples of the angiogenesis promoter include one kind or a mixture of more than one kind of the following substances: indigo carmine; 4- [N-methyl-N- (2-phenylethyl) amino] -1- (3, 5-dimethyl-4-propionyl aminobenzoyl) piperidine; 4- (5H-7, 8, 9, 10- tetrahydro-5, 7, 7, 10, 10-pentamethyl benz [e] naphtho [2, 3-b] [1,4] diazepine-13-yl) benzoic acid; activated protein C; urotensin II peptide compound; fibroblast growth factor (FGF) (including basicity FGF and acidity FGF); vascular endothelial cell growth factors (VEGF) (preferably a platelet-derived factor); hepatocyte growth factor (HGF); angiopoetin (including angiopoetin-1 and angiopoetin-2); platelet-derived growth factor (PDGF), Insulin-like growth factor (IGF) or smooth muscle embryo (SMemb). Of these substances, fibroblast growth factor is preferred (see, Hockel, M. et al., Arch. Surg., 128, 423, 1993). A basic fibroblast growth factor (bFGF) is preferred as a fibroblast growth factor, and a specific example includes trafermin (genetic recombination). Namely, a preferred embodiment of the gel-forming composition of the present invention is the above described gel-forming composition comprising trafermin, a pharmaceutically acceptable salt thereof, a pharmaceutically acceptable solvate thereof, or a prodrug thereof. "A pharmaceutically acceptable salt thereof" represents a salt of trafermin, and is specifically the same salt as above explained. "A pharmaceutically acceptable solvate thereof" represents a solvate of trafermin, and is specifically the same solvate as above explained. "A prodrug thereof" represents a prodrug of trafermin, and represents an agent that turns into trafermin, an ion thereof, or a pharmaceutically acceptable salt thereof, etc., in vivo after administration of a prodrug. In particular, a prodrug includes protecting groups such as an amino group which are taken off in vivo and it acts the same as trafermin.

### Anticancer Agent

Anticancer agent is a pharmaceutical agent for treating or preventing cancer. A publicly known anticancer agent can be used as needed. Specific examples of anticancer agent include the following substances: anticancer hemolytic streptococcus formulation such as OK-432 (commercial name Picibanil); anticancer polysaccharide such as krestin, lentinan, schizophyllan, and sonifilan; anticancer antibiotics such as mitomycin C (commercial name Mitomycin, etc.), actinomycin D (commercial name Cosmegen), bleomycin hydrochloride (commercial name Bleo), bleomycin sulfate (commercial name Bleo S), daunorubicin hydrochloride (commercial name Daunomycin), doxorubicin hydrochloride (commercial name Adriacin), neocarzinostatin (commercial name Neocarzinostatin), aclarubicin hydrochloride (commercial name Aclacinon), or epirubicin hydrochloride (commercial name Farmorubicin); mitotic inhibitor such as Vinblastine; alkylating agent such as cis-platin, carboplatin, and cyclophosphamide; antimetabolite such as 5-fluorouracil, cytosine arabinoside and hydroxyurea, N- {5- [N- (3, 4-dihydro-2-methyl-4-oxoquinazoline-6-ylmethyl) -N-methylamino] -2- thenoyl} -L-glutamic acid; anticancer antibiotics such as adriamycin and bleomycin; enzyme such as asparaginase; topoisomerase inhibitor such as etoposide; biological response modifier such as Interferon; antiestrogen such as "NOLVADEX" (tamoxifen); antiandrogen such as "CASODEX"; antimetabolite such as Fluorouracil, Tegafur, Tegafur-uracil, and Methotrexate; plant alkaloid such as Vncristine; anticancer antibiotic such as mitomycin C, actinomycin D, bleomycin hydrochloride, bleomycin sulfate, daunorubicin hydrochloride, doxorubicin hydrochloride, neocarzinostatin, aclarubicin hydrochloride, Aclacinon, and epirubicin hydrochloride; and platinum complex such as cyclotriphosphazene-platinum complex, and cisplatin-platinum complex.

A preferred embodiment of the gel-forming composition of the present invention is the above described gel-forming composition wherein the pharmaceutical agent is a gene. This gene is, for example, effective in treating and preventing a certain disease. A specific example of this gene is a calponin gene disclosed by patent No. 3713290. It is preferred that the gene be contained in the gel-forming composition as much as the effective amount for gene therapy. It is also preferred that the gene be contained in the gel-forming composition as it is (naked), in a micelle state, or in the form of a recombinant vector which is transformed into a known vector such as a virus vector. The pharmaceutical agent may be a known genetic antibody. A certain gene can be produced by a known production method generally used in the field of biology.

A preferred embodiment of the gel-forming composition of the present invention further comprises controlled-release formulation for controlling release of the pharmaceutical agent after gel formation is completed. The drug release controlling agent includes, for example, a metal ion containing solution to which a chitosan solution dropped (see Japanese Patent Laid-Open No. 2005-145873). Also, drug release controlling function can be achieved when the mixed amount of the pharmaceutically acceptable carrier is increased. When a known ligand is included in the gel-forming composition as a pharmaceutical agent, the receptor or the partial peptide of the receptor, for example, is preferred to be included as a drug release controlling agent. When the peptide has the characteristics of remaining in gel, ejection of the ligand is controlled, thereby achieving desirable sustained release.

A preferred embodiment of the gel-forming composition of the present invention is a gel-forming composition wherein the pharmaceutical agent is a ligand, or the gel-forming composition further comprises a ligand. Ligand is known to act as a blocker, an agonist, an antagonist or the like. So the gel-forming agent composition which includes an effective dose of known ligand as a pharmaceutical agent can be effectively used for preventing and treating ligand related diseases. Namely, the present invention can provide a gel-forming composition containing a ligand associated with a specific disease, and a therapeutic method for a specific disease using the gel-forming constituent. Also, since ligand has receptor-binding ability, when the pharmaceutical agent includes a receptor or a polypeptide having a steric structure, the same as that of a receptor, drug release can be suppressed by adding a ligand (having high affinity with gel) as a release controlling agent, thereby achieving desirable sustained release.

Known components such as a pharmaceutically acceptable carrier, other than gelator and diluent, may be added to the gel-forming composition as needed. Also, in order to control drug release time, the pharmaceutical agent itself of the present invention or a part of a pharmaceutical agent may be taken in a known pharmaceutically acceptable carrier, or the surface of a pharmaceutical agent may be coated. Specific examples of the coating agent are organic acid having chitosan and a reactive vinyl group and/or a polymer of the salt described in Japanese Patent Laid-Open No. 2002-087953.

### Drug Release Controlling Carrier

As above explained, in treating a bone defect disease or a bone deformation disease, it is desirable to maintain a constant drug release to the diseased site for prolonged period. However, if a pharmaceutical agent is administered with a gel-forming composition to treat a bone defect disease or a bone deformation disease in load-bearing joints (weight-bearing joints) such as hip joint, knee joint, ankle, or backbone, gels formed by the gel-forming composition suffer pressures from the joints. So if conventional gel-forming composition is used for drug administration, too much amount of drug is released too quickly and the drug release will not last long enough. On the other hand, as demonstrated in the examples described below, by using polyrotaxane or pseudo-polyrotaxane as a drug release controlling carrier of the gel-forming composition, desirable sustained drug release can be achieved in a diseased site even under a condition of continuous and repetitive pressure. Therefore, the drug release controlling carrier and the medical composition containing the carrier of the present invention can be used for treating bone/cartilage diseases in hip joint, knee joint, ankle, or backbone. In particular, the drug release controlling carrier and the medical composition containing the carrier of the present invention can be used for treating a bone/cartilage disease in knee joint (e.g., coxarthrosis). The bone/cartilage disease includes coxarthrosis or, defect or change in shape of a bone/cartilage caused by an injury.

Polyrotaxane comprises cyclic molecules and a linear molecule, wherein the linear molecule has the cyclic molecules included in a skewered manner, and sealing groups (i.e., stoppers, terminal groups) are arranged at both ends of the linear molecule for preventing the cyclic molecules from being separated. Polyrotaxane is, for example, introduced in "Applied Technology of Cyclic/Thecal Supermolecule New material", compiled by Toshikazu Takada and published by CMC Publication Ltd. In addition, Japanese Patent Laid-Open No. 2003-257488 discloses an example of a method for producing polyrotaxane. In the example, polyethylene glycol having amino groups at both ends, which is used as a chain macromolecule, is clathrated in an α-cyclodextrin. And then it is reacted with 2,4-dinitrofluorobenzene. Polyrotaxane thus obtained is bind with a sealing group by imino linkage.

On the other hand, a molecule whose cyclic molecule detaches from the linear molecule, for example, due to insufficient bulk of a sealing group is referred to as pseudo-rotaxane. And the molecule whose linear molecule has pluralities of cyclic molecules is referred to as pseudo-polyrotaxane. Examples of the pseudo-rotaxane and the pseudo-polyrotaxane, and an exemplary method for producing thereof are disclosed, for example, in Japanese patent No. 3704194. In the present invention, a pseudo-polyrotaxane is preferred for the use of a carrier of pharmaceutical agents.

Examples of cyclic molecules of the pseudo-rotaxane or the pseudo-polyrotaxane includes one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivatives thereof, pharmaceutically acceptable salts thereof, and pharmaceutically acceptable solvates thereof".

Examples of cyclodextrin derivatives include α-cyclodextrin, α-cyclodextrin, γ-cyclodextrin, dimethyl cyclodextrin, and glycosyl cyclodextrin.

The number of ring members of a crown ether molecule is not specifically limited, but is preferably 22 to 30, and more preferably 24 to 28. A ring of a crown ether molecule may have one or more than one of nitrogen atom, sulfur atom, phosphorus atom or the like as well as carbon atom and oxygen atom. Examples of crown ethers include dibenzo-24-crown-8, 24-crown-8, benzo-24-crown-8, bis (binaphthyl)-28-crown-8, bis (biphenyl)-28-crown-8, dicyclohexyl-24-crown-8, benzo/binaphthyl-24-crown-8, 1-aza-12-crown-4, 1-aza-15-crown-5, 1-aza-18-crown-6, 4,10-diaza-12-crown-4, 4,10-diaza-15-crown-5, and 4,13-diaza-18-crown-6. Out of these compounds, dibenzo-24-crown-8, 24-crown-8, benzo-24crown-8, or dicyclohexyl-24-crown-8 is preferred. Examples of benzo crown derivatives include benzo-12-crown-4, benzo-15-crown-5, benzo-18-crown-6, benzo-21-crown-7, benzo-24-crown-8, benzo-12-crown-4, benzo-15-crown-5, benzo-18-crown-6, benzo-21-crown-7, and 4'-nitrobenzo-15-crown-5.

Examples of dibenzo crown derivatives include dibenzo-12-crown-4, dibenzo-15-crown-5, dibenzo-18-crown-6, dibenzo-21-crown-7, dibenzo-24-crown-8, dibenzo-30-crown-10, and tribenzo-18-crown-6. Examples of cyclophane derivatives include octafluoro-[2, 2]paracyclophane disclosed in Japanese Patent Laid-Open No. 2001-213818, tetrafluoro-[2, 2]-paracyclophane disclosed in Japanese Patent Laid-Open No. 9-025252, dichloro-[2, 2]-paracyclophane disclosed in Japanese Patent Laid-Open No. 6-157372, dichloro tetrafluoro-[2, 2]-paracyclophane disclosed in Japanese Patent Laid-Open No. 9-059187, and 1, 1, 9, 9-tetrabromo-[2, 2]-paracyclophane disclosed in Japanese Patent Laid-Open No. 2000-080051.

Calixarene derivative is a generic term of compounds having a cyclic structure which couples phenol derivatives with alkylene groups or oxyalkylene groups. And a phenolic hydroxyl group in the molecule may be substituted with substituent groups.

As polyrotaxane or pseudo-polyrotaxane comprising cucurbituril and bipyridinium salt, the one disclosed in "Applied Technology of Cyclic/Thecal Supermolecule New material" compiled by Toshikazu Takada and published by CMC Publication Ltd. can be used as needed.

Examples of a chain molecule of a polyrotaxane or a pseudo-polyrotaxane includes one or more than one kind of chain molecules selected from a group of "polyalkylene glycol, copolymer of polyalkylene glycol, polyether, polyolefin, polyorganosiloxane, derivatives thereof, pharmaceutically acceptable salts thereof, and pharmaceutically acceptable solvates thereof".

Examples of "polyalkylene glycol" include polyethylene glycol and polypropylene glycol. Examples of "a copolymer of polyalkylene glycol" include polyethylene glycol and a copolymer of polypropylene glycol. The chain molecule, not restricted to chain macromolecules, may have branched chain. For example, it may be branched to 3 or more branches. It may also be substituted with substituent groups as needed. The molecular weight of the chain molecule is not specifically limited, but the number average molecular weight is preferably 400 to 500,000, and more preferably 2,000 to 200,000.

The ratio between the cyclic molecule and the chain molecule may be selected based on, for example, the characteristics of the resultant polyrotaxane. It is, for example, 100:1 to 1:100 in molar ratio.

The ratio between the chain molecule clathrated in cyclic molecules and the chain molecule not clathrated in cyclic molecules may be selected based on, for example, the characteristics of the resultant polyrotaxane. It is, for example, 100:1 to 1:100 in molar ratio.

As demonstrated in the example described below, pseudo-polyrotaxane comprising cyclodextrin, and polyethylene glycol, polypropylene glycol, or a copolymer of polyethylene glycol and polypropylene glycol is preferred as a drug release controlling carrier.

The drug release controlling carrier is preferably used for treating bone/cartilage disease such as bone deformity as well as treating bone defect sites. And since the drug release controlling carrier of the present invention can rather suppress drug release under conditions of continuous pressure, the carrier can be advantageously used for diseases of hip joint, knee joint, ankle or spine (knee joint in particular). Namely, the present invention can provide a pressure sensitive drug release controlling carrier. In particular, the present invention can provide a drug release controlling carrier which can maintain constant drug release by suppressing excessive drug release even under conditions of pressure.

As demonstrated in the example described below, there are preferred combinations between the drug release controlling agent and pharmaceutical agents to achieve desirable drug release. For example, a drug release controlling carrier containing pseudo-polyrotaxane comprising cyclodextrin, and polyethylene glycol, polypropylene glycol, or a copolymer of polyethylene glycol and polypropylene glycol exhibits particularly effective drug release when the carreir comprises, as pharmaceutical agents, thieno indazole derivative represented by below-described general formula (IV), prostaglandin A1 derivative, vitamin D derivative, vitamin K₂ derivative, eicosapentaenoic acid, benzyl sulfonic acid, bisphosphonic acid derivative, sex hormone derivative, phenol sulfo phthalein derivative, benzothiopyran or benzothiepine derivative, menatetrenone derivative, helioxanthine derivative, dihydrobenz [c] thiophene compound, tricyclic thiophene compound, Indian hedgehog (Ihh) or agonist of the signaling pathway thereof, PTH-related peptide or agonist of the signaling pathway thereof, pharmaceutically acceptable salts thereof, or pharmaceutically acceptable solvates thereof. Indian hedgehog (Ihh) is a secretory protein belonging to the hedgehog family. Secreted Ihh binds to Patched which is a receptor on a cell surface. Patched without Ihh suppresses Smoothened, but when Ihh binds to it, the suppression is taken off and signals are transmitted. Smoothened activates the Gli family which is a transcription factor, and Gli activates transcription of a target gene. PTHrP is a parathormone related peptide, and is a secretory protein. Secreted PTHrP binds to PTH/PTHRP receptors on cell surfaces. The receptor bound with the secreted PTHrP activates Gs and Gq which are intracellular G protein. Gs activates adenylate cyclase. Then cAMP is produced, and kinase such as protein kinase A is activated, thereby causing activation of the target gene. Gq activates PLC, thereby activating the target gene. Indian hedgehog, PTHrP, and an agonist of a signaling pathway thereof are disclosed, for example, in "hedgehog and chondrocytes" written by Shinsuke Ohba, Yuichi Chung, Kidney and Metabolic Bone Disease Vol.16 No.03 published by Japan medical center.

It is preferred that the amount of the drug release control carrier be optimally adjusted in accordance with diseases or its application. And the carrier may be not included in the gel-forming composition of the present invention, the amount of the carrier may be in the range from 0.1 % by weight to 80% by weight both inclusive, or the range may be from 1% by weight to 50% by weight both inclusive.

In the present invention, it is preferred that the dose of the pharmaceutical agents be adjusted depending on symptom, age, sex, administration method, etc. For on-site drug administration using the medical kit described below, it is preferred that one dosage be in the range from 0.005 mg/kg weight (preferably, 0.05 mg/kg weight) to 500 mg/kg weight (preferably, 50 mg/kg weight). It is preferred that the number of administration be once or a few times a day depending on symptom, but, since the gel formed from the gel-forming composition of the present invention has desirable sustained drug release, the number of administration may be only once, or may be once every month or every three months.

Trafermin, a pharmaceutical example, is commercially available as Fiblast (registered trademark). And the precautionary statement of the Fiblast indicates that 30 µg of trafermin should be administered once a day by injecting trafermin solution of 100 µg/ml concentration. When the gel-forming composition of the present invention includes trafermin as a pharmaceutical agent, and is used as a wound coating material, it is preferred that the gel be sprayed on a wound or an ulcer by the drug administration device of the present invention having spray ports. In this case, the administration amount will be about the same as the above described administration amount. In particular, since the gel composition of the present invention has desirable sustained drug release, it is preferred that the gel composition having 0.5 g to 5 g of trafermin be administered once (or once a month).

The strength of the gel formed from the gel-forming compositions of the present invention is not specifically limited, but as is demonstrated in an example described below, it is from 0.3 N/cm² to 3 N/cm², preferably from 1 N/cm² to 2 N/cm², an more preferably from 1.2 N/cm² to 2 N/cm². Especially when the gel-forming composition of the present invention is administered on-site to bone defect sites, etc., it is desired that the gel have adequate strength. And since the gel has a desired strength, the gel-forming composition of the present invention can act as, for example, a bone filler effectively. As demonstrated in the examples described below, the strength of the gel can be adjusted by selecting kind, concentration, and pH of gelator and solvent as needed. Note that the term "gel strength" in this specification represents a value shown by the load (N/cm²) when a rod (the diameter of 2 mm) of a load meter entered into 98% of the height of a gel sample.

### Major Application of the Gel-forming Composition of the Present Invention

The gel formed from the gel-forming composition of the present invention has the characteristics of particularly effective for treating defect bone sites. So the gel-forming composition of the present invention, for example, can be effectively used as a pharmaceutical composition such as a composition for treating osteoarthritis. In particular, two components are mixed immediately before the administration, and then the mixed solution is administered to the affected area. The two components may also be administered directly on site. Also, the gel-forming composition of the present invention can be effectively used as a wound coating composition by being administered directly to a tumor or an affected area. Furthermore, the present invention can provide a predetermined pharmaceutical agent for processing a gel-forming composition comprising the first composition and the second composition.

### Administration Device of the Present Invention

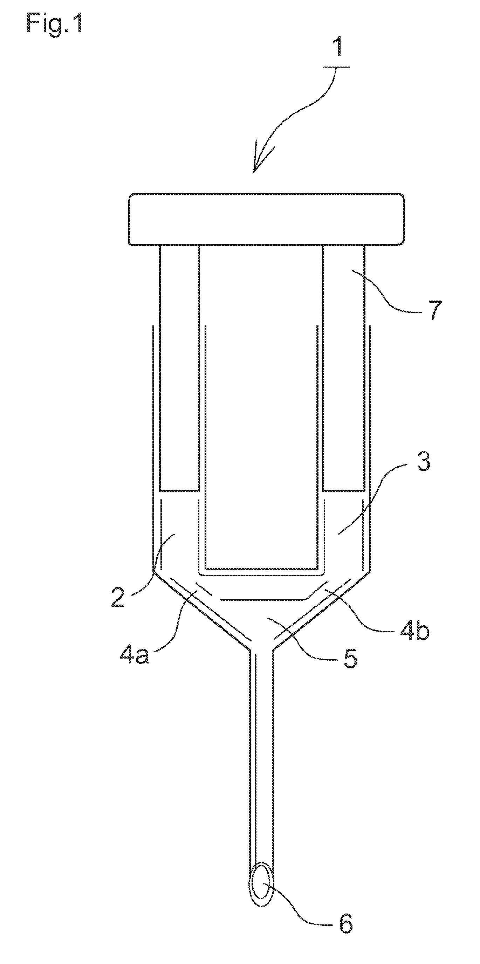

Fig.1 is a schematic diagram showing the administration device of the present invention. The administration device of the present invention is basically an above described administration device (1) containing the gel-forming composition. The administration device (1) comprises: two containing rooms (2, 3) containing the first composition and the second composition and spatially isolating the compositions from each other; moving parts (4a, 4b) being connected to the two containing rooms (2, 3), the first composition and the second composition respectively being able to move therethrough; a mixing part (5) mixing the first composition and the second composition which have moved through the moving parts (4a, 4b); and a discharge part (6) discharging the first composition and the second composition which have been mixed in the mixing part (5). Reference number 7 in Fig.1 is a syringe for pushing out the compositions from the accommodation rooms to the move parts by putting pressure on the compositions.

The operational example of the above described administration device is described below. When the syringe (7) is pressed by fingers, the compositions accommodated in the accommodation room (2, 3) are pushed to the moving parts (4). When the syringe is pressed further, the compositions moves into the mixing part from the moving parts, and the two compositions are mixed. Then, the mixture of these compositions is gelated in the mixing part. One of the compositions may be gel state, or the mixture may be gelated after having been administered to the affected area. In general, gelation begins about one minute after the two compositions having been mixed. So when the syringe (7) is pressed, a liquid mixture of the two compositions is administered to the affected area. On the other hand, if gelation begins immediately after the two compositions have been mixed, or if gelation is in progress after the two compositions have been mixed, when the syringe (7) is pressed, mixture of the two compositions gelated in the mixing part (5) is administered to the affected area such as bone defect site from the discharge part (6). In this case, however, controlling the gel state suitable for administration requires high technique. So as above stated, when an administration device having the mixing part (5) is employed, it is preferred to use a gel-forming composition whose gelation begins about one minute after the two compositions have been mixed rather than a gel-forming composition whose gelation begins immediately after the two compositions have been mixed. Hereinafter, each component of the administration device of the present invention is described.

### Containing Room

The two containing rooms (2, 3) contain the first composition and the second composition, and spatially isolate the components from each other. The containing rooms are not specifically limited, if they have enough volume to contain the first composition and the second composition. The volume of each containing room is, for example, 0.5 mL to 10 mL.

### Moving Part

The moving parts (4a, 4b) are connected to the two containing rooms (2, 3). And the first composition and the second composition respectively moves therethrough to the mixing part. The containing part may be connected directly to the mixing part.

### Mixing Part

In the mixing part (5), the first composition and the second composition which have moved through the moving parts (4a, 4b) are mixed. If the volume of the mixing part (5) is large, large amount of the two compositions are mixed in the mixing part, thereby inducing gelation within the mixing part. So the volume of the mixing part is preferably 0.1 mL to 10 mL, more preferably 0.5 mL to 1 mL.

### Discharge Part

The discharge part (6) discharges the first composition and the second composition which have been mixed in the mixing part (5). As the injection needle connecting between the mixing part (5) and the discharge part (6), a large-bore needle is preferred because, in some cases, the gelation proceeds within the mixing part and the viscosity of the liquid mixture rises. The specific bore diameter of the needle is preferably 0.5 mm to 3 mm, more preferably 1 mm to 2 mm. The discharge part (6) is preferred to have a spray port. The administration device having a spray port can spray liquid composition on an affected area.

### Syringe

A known syringe used for a general injection syringe can be used as the syringe (pressuring part (7)) as needed.

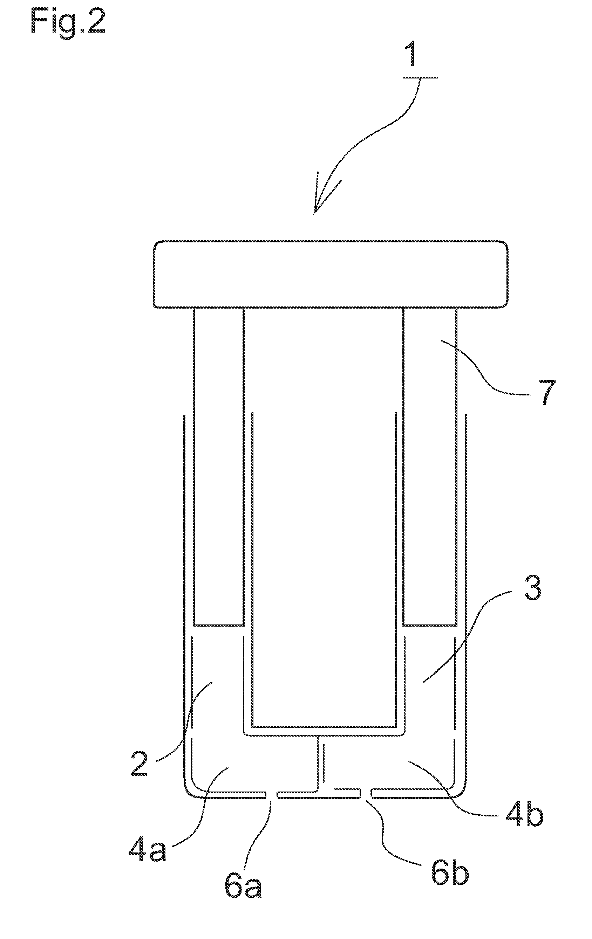

Another preferred embodiment of the administration device of the present invention comprises: a mixing part (5) mixing the first composition and the second composition which have moved through the moving parts (4a, 4b); and discharge parts (6a, 6b) discharging the first composition and the second composition separately which have moved through the moving parts (4a, 4b) in place of the discharge part (6) discharging the first composition and the second composition which have mixed together in the mixing part (5). This administration device, as shown in Fig. 2, comprises: two containing rooms (2, 3) containing the first composition and the second composition and spatially isolating the compositions from each other; and moving parts (4a, 4b) being connected to the two containing rooms (2, 3), the first composition and the second composition respectively being able to move therethrough; and discharge parts (6a, 6b) discharging the first composition and the second composition separately which have moved through the moving parts (4a, 4b).

The administration device according to this embodiment can mix the two compositions on site. For a gel-forming composition which begins gelation immediately after the two compositions have been mixed, the administration device according to this embodiment can be advantageously used. Furthermore, since the administration device according to this embodiment does not have the mixing part, the device can prevent gel-forming compositions from forming a gel.

It is preferred that the discharge parts (6a, 6b) of the administration device according to this embodiment have spray ports. The administration device having this type of spray ports can spray liquid composition on an affected area. Namely, the sprayed two compositions are mixed on site, and begin gelation. Since the two compositions are sprayed, even though the two compositions are administered separately, the two compositions are mixed on the administered area, and the gelation is promoted.

The present invention can also provide a therapeutic method of treating bone/cartilage disease such as osteoarthritis using the gel-forming composition or the administration device of the present invention.

### Medical Kit

The present invention can also provide the above described gel-forming composition and a medical kit comprising the above described administration device. The medical kit specifically comprises: a pharmaceutical agent; a first gelator comprising one or more than one kind of a compound represented by the general formula (I) or (II), or a compound including 3 to 8 repeating units represented by the general formula (III); a second gelator comprising one or more than one kind of a compound represented by the general formula (I) or (II), or a compound including 3 to 8 repeating units represented by the general formula (III); a diluent; and the administration device. The medical kit of the present invention may comprise agents other than the above described ones as needed. The pharmaceutical agent, the first gelator, and the second gelator may be separately contained in the other containers, or may be mixed together as needed. Furthermore, the pharmaceutical agent may be mixed with the diluent; the pharmaceutical agent, the first gelator, and the diluent may be mixed together; the second gelator and the diluent may be mixed together; or the pharmaceutical agent, the second gelator, and the diluent may be mixed together. Also, the above described stabilizing agent may be contained in the medical kit. The stabilizing agent may be mixed with either one of the pharmaceutical agent, the first gelator, and the diluent, or it may be contained apart from these agents.

A preferred embodiment of the medical kit of the present invention comprises: a containing room (2) where the pharmaceutical agent, the first gelator, and the diluent are contained; and another containing room (3) where the second gelator, and the diluent are contained. This medical kit can administer the pharmaceutical agent and the gel-forming composition easily by pushing the syringe aiming the discharge part (6) at an affected site.

When the chemical compound contained in the gel-forming agent is prone to be hydrolyzed, e.g., an ester compound, it is preferred that a medical kit which contains gelator and diluent separately be used so that they are mixed immediately before the gel-forming agent is administered.

The kit of the present invention can administer gel which contains a pharmaceutical agent directly to an administration site such as a cartilage defect site. The kit of the present invention, used with an endoscope etc., also enables administration of the pharmaceutical agent directly to the object site not being accompanied by oral administration, nor cell transplantation, thereby providing a low invasive treatment. Namely the medical kit of the present invention together with the gel of the present invention acts as an excellent DDS.

### Example 1

### Preparation of Two-Component On Site Hardening Gel Composition

In this example, in order to examine the properties of gel compositions, two-component gel compositions were produced. In first component (gel composition), pentaerythritol tetra polyethylene glycol ethers propylamine ("4PA-PEG10,000") of weight average molecular weight 10000 was dissolved in predetermined solvents at a concentration of 10mM.

In second component, pentaerythritol tetra polyethylene glycol ethers succinimidylglutarate ("4GS-PEG10,000") of weight average molecular weight 10000 was dissolved in predetermined solvents at a concentration of 10 mM.

In this example, the same solvent medium were used for the first component and the second component. In particular, the following solvents were used: pure water; phosphate buffer solution of pH 6, pH 7.4, and pH 9; citrate buffer solution of pH 6, pH 7.4, and pH 9; phosphate buffered saline (PBS); and physiologic saline solution. The pH of the phosphate buffer solution was adjusted to desired pH by mixing 200 mM sodium dihydrogen phosphate with 200 mM disodium hydrogen phosphate. The citrate buffer solution was adjusted to desired pH by mixing 200 mM citric acid with 200 mM trisodium citrate. The phosphate buffered salin was adjusted to be around pH 7.4 by mixing 137 mM sodium chloride, 8.1 mM disodium hydrogen phosphate, 2.68 mM potassium chloride, and 1.47 mM potassium dihydrogen phosphate. The isotonic sodium chloride solution used was "Otsuka Sodium Chloride Injection (The Japanese Pharmacopeia)" manufactured by Otsuka Pharmaceutical Co., Ltd.

In this example, the first component was prepared before the second component was prepared. And the second component was prepared immediately before use. This is because the solution of 4GS-PEG10000 which is the second component is unstable in aqueous solvent.

### Example 2

### Preparation for Two-Component On Site Hardening Gel Composition

In this example, in order to examine the properties of gel compositions, two-component gel compositions were produced. In first component, pentaerythritol tetra polyethylene glycol ethers propylamine ("4PA-PEG20,000") of weight average molecular weight 20000 and 2 functionality propylamine - polyethylene glycol ("2PA-PEG5, 000") of weight average molecular weight 5000 were dissolved in 20 mM phosphate buffer solution of pH 7.4 at a concentrations of 10 mM and 20 mM, respectively.

In second component, pentaerythritol tetra polyethylene glycol ethers succinimidylglutarate ("4GS-PEG20,000") of weight average molecular weight 20000 and 2 functionality succinimidylglutarate - polyethylene glycol ("2GS-PEG5,000") of weight average molecular weight 5000 were dissolved in 20 mM phosphate buffer solution of pH 7.4 at a concentrations of 10 mM and 20 mM, respectively.

In this example, the first component was prepared before the second component was prepared. And the second component was prepared immediately before use. This is because mixture solution of 4GS-PEG20,000 and 2GS-PEG5,000 which is the second component is unstable in aqueous solvent.

### Example 3

### Verification of Gel Strength and Gelation Time in Accordance with Diluents

In accordance with the Example 1, two-component solutions were prepared by dissolving 4PA-PEG10,000 and 4GS-PEG10,000 in predetermined solvents at a concentration of 10 mM, respectively. The predetermined solvents included pure water, 20 mM phosphate buffer solution of pH 7.4, PBS of pH 7.4, and physiologic saline solution. In this way, 0.5 mL of each solvent was prepared. Right after the two-component solutions were prepared, they were mixed with each other in a 50 mL tube manufactured by BD Falcon Co., Ltd. Then, the mixture were gelated at 37ºC and the gel strengths were measured.

On the other hand, control gels, 0.5% carrageenin - 0.5% locust bean gum, 1.0% carrageenin - 1.0% locust bean gum, and 2.0% carrageenin - 2.0% locust bean gum, which are gelators for food, were prepared. In particular, each powder of gelators is weighted and mixed. And then, 1.0 mL of 20 mM phosphate buffer solution of pH 7.4 was added to the mixture of the powders, and the solutions were warmed at 80ºC in warming baths and the powders were dissolved. After the powders were dissolved, the solutions were gelated and the gel strengths were measured.

The gel strengths were measured by the load (N/cm²) when a rod (the diameter of 2 mm) of a load meter entered into 98% of the height of gel samples. Also, the gel rupture and deformation rate was measured by gel deformation rate when a rod (the diameter of 2 mm) of a load meter entered into until the deformation rate was 100%. The results were shown in the table 1 below.

**[Table 1]**

| Relationship between diluents added and gel strength | | |
|---|---|---|
| Gel component (upper) Diluent (lower) | Gel strength (N/cm²) | Gel rupture and deformation rate (%) |
| 4PA-PEG 10,000 and 4GS-PEG 10,000 20 mM phosphate buffer solution, pH 7.4 | 1.66 | No gel rupture |
| 4PA-PEG 10,000 and 4GS-PEG 10,000 PBS, pH 7.4 | 1.22 | No gel rupture |
| 4PA-PEG 10,000 and 4GS-PEG 10,000 Pure water | 0.67 | No gel rupture |
| 4PA-PEG 10,000 and 4GS-PEG 10,000 Physiologic saline solution | 0.45 | No gel rupture |
| 0.5% carrageenin and 0.5% locust bean gum 20 mM phosphate buffer solution, pH 7.4 | 0.019 | 20.8 |
| 1.0% carrageenin and 1.0% locust bean gum 20 mM phosphate buffer solution, pH 7.4 | 0.047 | 46.2 |
| 2.0% carrageenin and 2.0% locust bean gum 20 mM phosphate buffer solution, pH 7.4 | 0.166 | 36.2 |

It can be seen from the table 1 that the gels with high strength can be obtained by the two-component gel compositions of the present invention even mixed with aqueous solvents. Particularly, it can be seen that a gel with high strength can be obtained by using 20 mM phosphate buffer solution of pH 7.4 as a diluent. It can also be seen that the two-component gel compositions of the present invention has high viscoelasticity and gel strength because the composition suffers no fracture even when a rod (the diameter of 2 mm) of a load meter entered into until the deformation rate was 100%. Therefore, it can be seen that the two-component gel compositions of the present invention have characteristics of bearing load that the compositions suffer during treatment. When pure water, 20 mM phosphate buffer solution of pH 7.4, and PBS of pH 7.4 were used as diluents, gelation begun in a minute after the two components were mixed, and firm gels were formed in three minutes. On the other hand, when isotonic sodium chloride solution was used as a diluent, a hard gel was formed within five minutes.

### Example 4

### Verification of Gel Strength and Gelation Time in Accordance with the pH of Diluents

In accordance with the Example 1, two-component solutions were prepared by dissolving 4PA-PEG10,000 and 4GS-PEG10,000 in predetermined solvents at a concentration of 10 mM, respectively. The predetermined solvents included 20 mM phosphate buffer solution of pH 6, pH 7.4, and pH 9, and 20 mM citrate buffer solution of pH 6, pH 7.4, and pH 9. In this way, 0.5 mL of each solvent was prepared. Right after the two solutions were prepared, they are mixed with each other in a 50 mL tube manufactured by BD Falcon Co., Ltd. Then, the mixtures were gelated at 37ºC and the gel strengths were measured. The results were shown in the table 2 below.

**[Table 2]**

| Relationship between pH of diluents and gel strength | |
|---|---|
| Diluent | Gel strength (N/cm²) |
| 20 mM phosphate buffer solution, pH 6.0 | 0.952 |
| 20 mM phosphate buffer solution, pH 7.4 | 1.66 |
| 20 mM phosphate buffer solution, pH 9.0 | 1.42 |
| 20 mM citrate buffer solution, pH 6.0 | 0.86 |
| 20 mM citrate buffer solution, pH 7.4 | 1.29 |
| 20 mM citrate buffer solution, pH 9.0 | 1.02 |

It can be seen from the table 2 that the gel strengths were maximized when diluents of around pH 7.4 were used for both cases. When phosphate buffer solution of pH 7.4 and pH 9 or citrate buffer solution of pH 7.4 and pH 9 were used as diluents, the gelation begun in a minute after the two components were mixed, and firm gels were formed in three minutes. On the other hand, when phosphate buffer solution of pH 6 and citrate buffer solution of pH 6 were used as diluents, hard gels were formed within five minutes.

### Example 5

### Verification of Gel Strength and Gelation Time in Accordance with the Concentration of the Diluent

In accordance with the Example 1, two-component solutions were prepared by dissolving 4PA-PEG10,000 and 4GS-PEG10,000 in predetermined solvents at a concentration of 10mM respectively. The predetermined solvents included 2 mM, 20 mM, 100 mM, and 200 mM phosphate buffer solution of pH 7.4, and 2 mM, 20 mM, 100 mM, and 200 mM citrate buffer solution of pH 7.4. In this way, 0.5 mL of each solvent was prepared. Right after the two solutions were prepared, they were mixed with teach other in a 50 mL tube manufactured by BD Falcon Co., Ltd. Then, the mixtures were gelated at 37ºC and the gel strengths were measured. The results were shown in the table 3 below.

**[Table 3]**

| Relationship between concentration of diluents and gel strength | |
|---|---|
| Diluent (pH 7.4) | Gel strength (N/cm²) |
| 2 mM phosphate buffer solution | 0.67 |
| 20 mM phosphate buffer solution | 1.66 |
| 100 mM phosphate buffer solution | 1.57 |
| 200 mM phosphate buffer solution | 1.30 |
| 2 mM citrate buffer solution | 0.63 |
| 20 mM citrate buffer solution | 1.29 |
| 100 mM citrate buffer solution | 0.88 |
| 200 mM citrate buffer solution | 0.85 |

It can be seen from the table 3 that the gel strengths were maximized when phosphate buffer solution of around 20 mM and citrate buffer solution of around 20 mM were used as diluents. Regardless of the concentration of the diluents, gelation of all the solutions begun in one minute after the two components were mixed, and hard gels were formed within three minutes.

### Example 6

### Verification of Gel Strength and Gelation Time in Accordance with Salt Concentration of Diluents

In accordance with the Example 1, two-component solutions were prepared by dissolving 4PA-PEG10,000 and 4GS-PEG10,000 in predetermined solvents at a concentration of 10mM, respectively. The predetermined solvents included pure waters whose salt concentration were adjusted respectively to 0 mM, 50 mM, 100 mM, and 200 mM, and 20 mM phosphate buffer solution of pH 7.4. In this way, 0.5 mL of each solvent was prepared. Right after the two solutions were prepared, they were mixed with each other in a 50 mL tube manufactured by BD Falcon Co., Ltd. Then, the mixtures were gelated at 37ºC and the gel strengths were measured. The results were shown in the table 4 below.

**[Table 4]**

| Relationship between salt concentration of diluents and gel strength | | |
|---|---|---|
| Diluent | NaCl concentration (mM) | Gel strength (N/cm²) |
| Pure water | 0 | 0.62 |
| Pure water | 50 | 0.71 |
| Pure water | 100 | 0.56 |
| Pure water | 200 | 0.57 |
| 20 mM phosphate buffer solution, pH 7.4 | 0 | 1.66 |
| 20 mM phosphate buffer solution, pH 7.4 | 100 | 1.34 |
| 20 mM phosphate buffer solution, pH 7.4 | 200 | 1.38 |
| 20 mM phosphate buffer solution, pH 7.4 | 400 | 1.12 |

It can be seen from the table 4 that high strength gels can be obtained in a wide range of salt concentrations.

### Example 7

### Verification of Gel Strength and Gelation Time in Accordance with the Molecular Weight and the Structure of Gelator Agents

In accordance with the Example 1 or the Example 2, two-component solutions were prepared by dissolving predetermined gelators in a predetermined solvent. The predetermined gelators were as shown in table 5 below. The concentration of 2PA-PEG5000 was 20 mM, and the rest were 10 mM. 20 mM phosphate buffer solution of pH 7.4 was used as the predetermined solvent. In this way, 0.5 mL of each solvent was prepared. Right after the two-component solutions were prepared, they are mixed with each other in a 50 mL tube manufactured by BD Falcon Co., Ltd. Then, the mixtures were gelated at 37ºC and the gel strengths were measured. The results were shown in the table 5 below.

**[Table 5]**

| Gel strength and gelation time in accordance with the molecular weight and the structure of gelator agents | | | |
|---|---|---|---|
| First gelator | Second gelator | Gel strength (N/cm²) | Gelation time |
| 4PA-PEG 10,000 | 2GS-PEG 5,000 | 0.71 | Gelated in 5 minutes |
| 2PA-PEG 5,000 | 4GS-PEG 20,000 | 0.14 | Gelated in 5 minutes |
| 4PA-PEG 10,000 | 4GS-PEG 10,000 | 1.66 | Started gelation in 1 minute Gelated in 3 minutes |
| 4PA-PEG 10,000 | 4GS-PEG 20,000 | 0.89 | Started gelation in 1 minute Gelated in 3 minutes |
| 4PA-PEG 20,000 | 4GS-PEG 20,000 | 1.69 | Started gelation in 1 minute Gelated in 3 minutes |

It can be seen from the table 5 that gels with excellent gel strength and gelation time can be obtained by mixing two components which are mixtures of various structures based on PEG (4-arm PEG and 2-arm PEG) and molecular weights (5,000, 10,000, 20,000). Also, it can particularly be seen that the gel formed by mixing a component containing 4PA-PEG10,000 and a component containing 4GS-PEG10,000, and the gel formed by mixing a component containing 4PA-PEG20,000 and a component containing 4GS-PEG20,000 have characteristics of excellent gel strength and gelation time. Although the molecular weight of gelator is not specifically limited, it can be seen that preferred gels can be obtained by using gelators having molecular weights of 3000 to 40000, and more preferred gels can be obtained by using gelators having molecular weights of 10000 to 30000.

### Example 8

### Verification of Influence on Gel Strength and Gelation Time in Accordance with the Concentration of a Gelator

In accordance with the Example 1, two-component solutions were prepared by dissolving 4PA-PEG10,000 and 4GS-PEG10,000 in predetermined solvents at a concentration of 5 mM, 10 mM, and 20 mM, respectively. The predetermined solvents were phosphate buffer solutions of pH 7.4. In this way, 0.5 mL of each solvent is prepared. Right after the two solutions were prepared, they are mixed with each other in a 50 mL tube manufactured by BD Falcon Co., Ltd. Then, the mixtures were gelated at 37ºC and the gel strengths were measured. The results are shown in Table 6 below.

**[Table 6] Concentration of gelator and gel strength**

| Concentration of gelator (mM) | Gel strength (N/cm²) |
|---|---|
| 5 | 0.97 |
| 10 | 1.93 |
| 20 | 1.67 |

The mixtures whose gelator concentrations were 10 mM and 20 mM begun gelated in a minute after the two components were mixed, and firm gels were formed within three minutes. On the other hand, the mixture whose gelator concentration was 5 mM formed a firm gel within five minutes after the two components were mixed. It can be assumed from these results that a preferred gel can be formed when the concentration of gelator is in between 5 mM to 20 mM. It can also be estimated that a desirable gel can be formed when the concentration of gelator is in between 6 mM to 30 mM.

### Example 9

### Verification of Stability and Mechanical Characteristic Change of the Gel of the Present Invention in a Solution

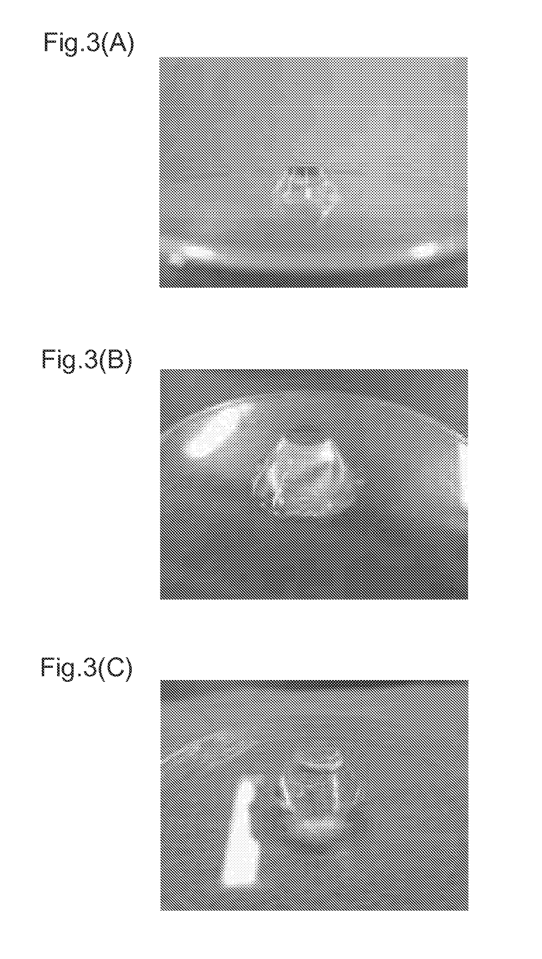

In accordance with the Example 1, two-component solutions were prepared by dissolving 4PA-PEG10,000 and 4GS-PEG10,000 in predetermined solvents at a concentration of 10mM, respectively. The predetermined solvents were 20 mM phosphate buffer solutions of pH 7.4. In this way, 0.5 mL of each solvent is prepared. Right after the two solutions were prepared, they are mixed with each other in a 50 mL tube manufactured by BD Falcon Co., Ltd. Then, the mixture was gelated at 37ºC. The gel was set in PBS, and characteristics change with time thereof was observed. The results are shown in Fig. 3. Fig. 3 shows photographs, in place of a diagram, showing temporal changes of the gel in PBS. Fig. 3(A) shows the gel one week later. Fig. 3(B) shows the gel fourteen weeks later. Fig. 3(C) shows the gel twenty eight weeks later. It can be seen from the Fig. 3 that the gel obtained by the gel-forming composition of the present invention can maintain its form for a long time.

Gel rupture and deformation rate measured immediately after the gel formation was 140%, which was high viscoelasticity. Even after the gel was administered in PBS, it maintained its gel rupture and deformation rate of over about 110%, and even one month after the administration, the gel rupture and deformation rate was maintained at high viscoelasticity level of over about 120%. Furthermore, stable form of the gel was maintained without being dissolved even two months after the gelation. From these results, the gel of the present invention can maintain its form as well as high viscoelasticity in vivo for long time. Therefore, the gel of the present invention is a preferred gel that can endure with chronic therapy such as cartilage treatment.

### Example 10

### Verification of Sustained Release of a Chondrogenesis Promoting Factor TM of the Gel of the Present Invention

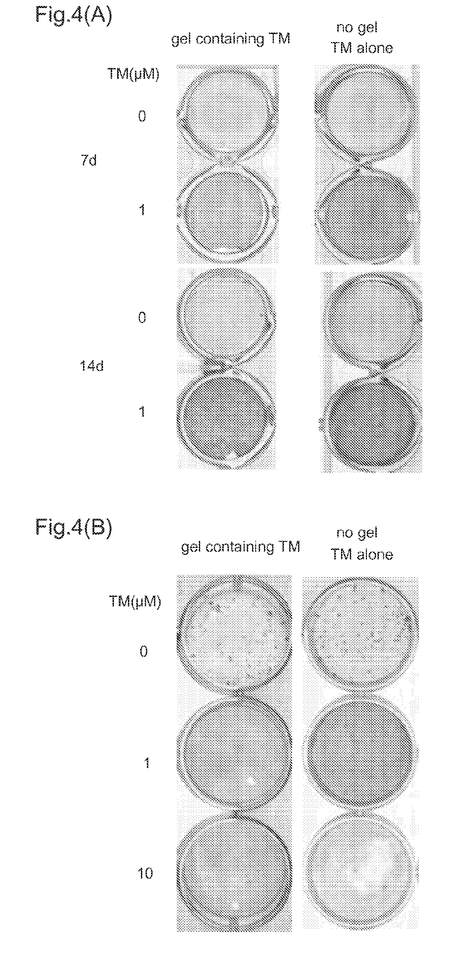

In accordance with the Example 1, two-component solutions were prepared by dissolving 4PA-PEG10,000 and 4GS-PEG10,000 in predetermined solvents at a concentration of 10mM, respectively. The predetermined solvents were 20 mM phosphate buffer solutions of pH 7.4. TM was added to the first gel component at a TM concentration of the gel liquid mixture of 1 µM or 10µM. Also, a control which does not contain TM was prepared. In this way, 0.5 mL of each solvent is prepared. Right after the two solutions were prepared, they are mixed with each other in a 50 mL tube manufactured by BD Falcon Co., Ltd. Then, the prepared liquid mixture containing TM was administered to C3H10T1/2 (undifferentiated mouse embryo mesenchymal cell) cell culture medium. One week and two weeks after the gel administration, cartilage matrix expression levels were evaluated by toluidine blue staining. Fig. 4 shows photographs, in place of a diagram, showing the results of toluidine blue staining for examining sustained release of the chondrogenesis promoting factor TM in the gel of the present invention. Fig. 4(A) shows the results of toluidine blue staining using TM concentrations (0 µM and 1 µM), 7 days (7d) or 14 days (14d) had elapsed after the gelation is completed. Fig. 4(B) shows the results of toluidine blue staining using TM concentrations (0.1 µM and 10 µM) one week has elapsed after the gelation is completed.

Fig. 4 demonstrated that cells to which gels containing TM of 1 µM and 10 µM were administered expressed cartilage matrices one week and two weeks after the gel administration. On the other hand, the control (the gel not containing TM) did not express cartilage matrix. Also, when TM of 10 µM was administered directly to the cell culture medium by a syringe, the cells died. However when TM of 10 µM was administered together with the gel composition of the present invention, the cells did not die and the expression of cartilage matrix was verified. Therefore, the gel of the present invention not only acts as an effective DDS but also has sustained release of pharmaceutical agents such as chondrogenesis promoter (e.g., TM). As above mentioned, when TM is administered directly to the cells, they die, and when TM of the same concentration being contained by the gel of the present invention is administered, the cells differentiate into chondrocyte without perishing. It is considered that this is because the gel of the present invention has sustained release thereby releasing the pharmaceutical agents gradually with adequate emission concentration and velocity not releasing the pharmaceutical agents contained by the gel all together. These properties are quite effective in administrating pharmaceutical agents for cartilage disease treatment and the like.

### Example 11

### Verification of Durability of Sustained Release of Chondrogenesis Promoting Factor TM of the Gel of the Present Invention

Two components were prepared in the same way as the Example 10. Two components were mixed, and then the mixture was administered to C3H10T1/2 (undifferentiated mouse embryo mesenchymal cell) cell culture medium. Then, in four days interval, the gel was transferred to the other cell culture medium, and durability of the sustained release of TM was verified. Differentiation to chondrocyte was verified in both gels containing TM of 1 µM and 10 µM. However, after two weeks from the administration, higher level of cartilage matrix expression was observed in the C3H10T1/2 cell culture medium which was administered the gel containing TM of 10 µM than that which was administered the gel containing TM of 1 µM. Also, the gel containing TM of 10 µM exhibits preferred sustained release of TM even one month after the administration, and chondrocyte differentiation of the gel administered cell was verified. The results show that the gel of the present invention has sustained release, thereby releasing the pharmaceutical agents gradually with adequate emission concentration and velocity for a long period. These properties are quite effective in administrating pharmaceutical agents for cartilage disease treatment and the like.

### Example 12

### Verification of Gelation of Two Components Using a Two-Component Mixing Syringe

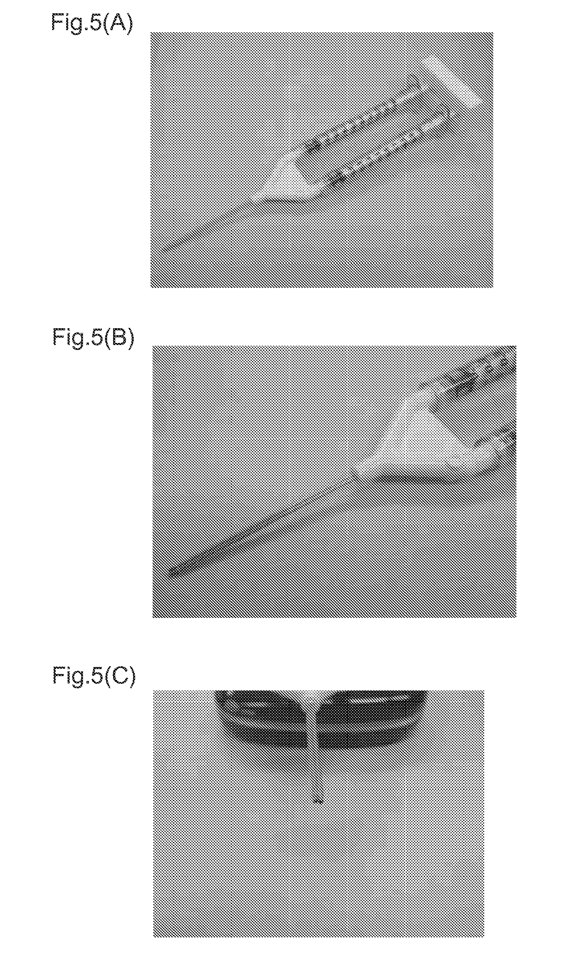

Two components were prepared in the same way as the Example 10. Two components were mixed, and then the mixture was immediately filled in a two-component mixing syringe. Fig.5 shows the two-component mixing syringe of this embodiment. Fig. 5 shows photographs, in place of a diagram, showing the two-component mixing syringe (the device of the present invention). Fig. 5(A) shows the overall view. Fig. 5(B) shows the vicinity of the moving part. Fig. 5(C) shows the vicinity of the discharge part. The administration device of this embodiment does not include the mixing part (5). And the two components are not mixed in the moving part shown in Fig. 5(B). Although, an administration device in which two components are mixed can easily be manufactured in the same way as this administration device is manufactured. The two components are mixed by applying pressure on the two-component mixing syringe, and the liquid mixture was put out on a plastic petri dish. The liquid mixture begun gelation in a minute, and a firm gel formed within 3 minutes. So the gel of the present invention can be easily administered to desired administration sites by the medical kit of the present invention including the two-component mixing syringe. For example, the medical kit of the present invention can administer a gel containing pharmaceutical agents directly to a drug administration site such as a cartilage defect site. The kit of the present invention, used with an endoscope etc., enables administration of pharmaceutical agents directly to an object site not being accompanied by oral administration, thereby providing a low invasive treatment. Namely the medical kit of the present invention together with the gel of the present invention acts as an excellent DDS.

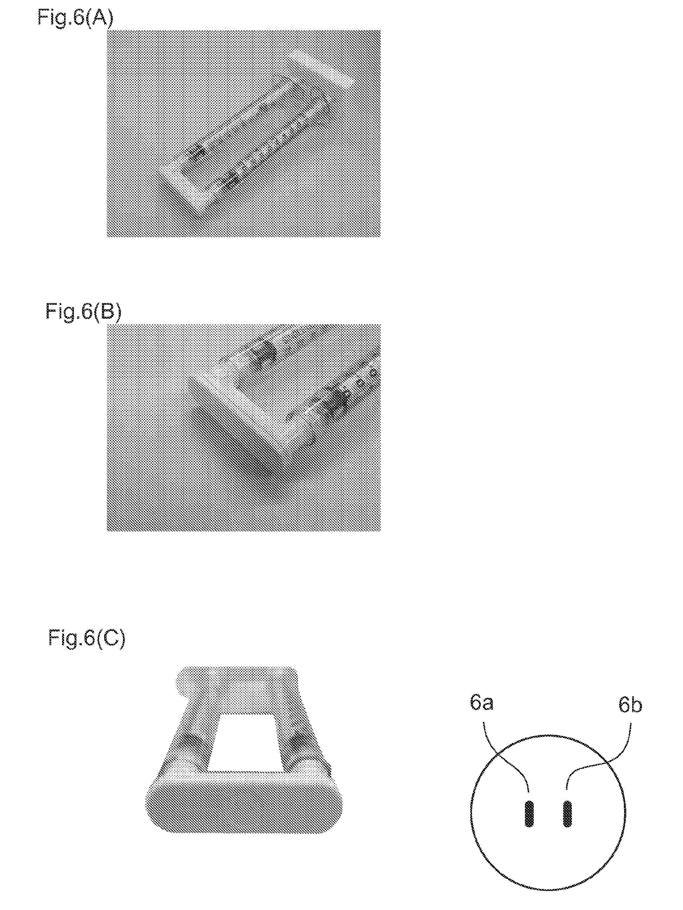

Fig. 6 shows an administration device having spray ports at the discharge parts (6a, 6b). Fig. 6 shows photographs, in place of a diagram, showing the administration device having two spray ports at the discharge parts. Fig. 6(A) shows the overall view. Fig. 6(B) shows the vicinity of the moving parts (4a, 4b). Fig. 6(C) shows the vicinity of the discharge parts. Fig. 6(C) includes a figure of the discharge parts to promote understanding thereof in addition to the picture. The administration device of this embodiment does not include a mixing part (5). And two components are not mixed in the moving parts shown in Fig. 6(B). Although, an administration device in which two components are mixed can easily be manufactured in the same way as this administration device is manufactured.

### Example 13

### On-Site Hardening Gel DDS in Pig's Knee Cartilage Defect Model

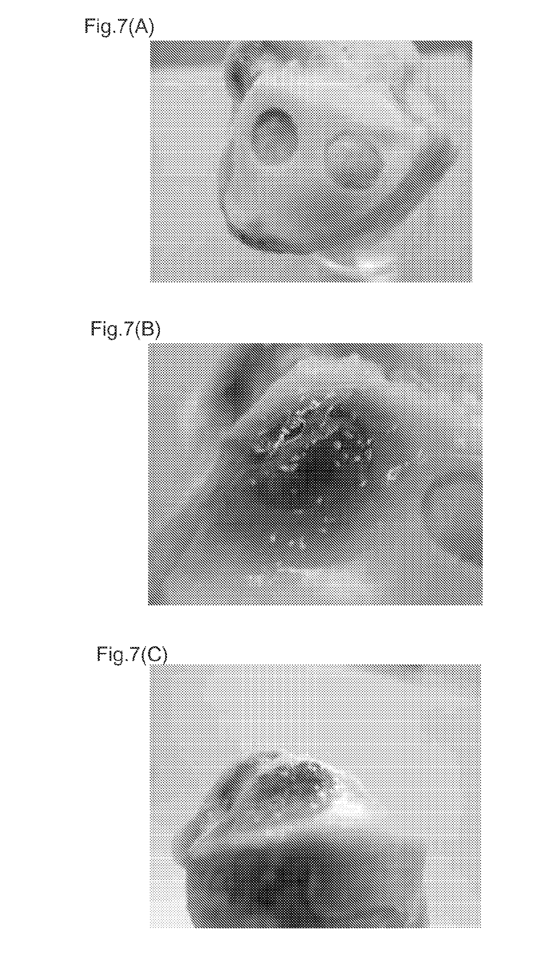

Fig. 7 explains the present example. Fig. 7 shows photographs, in place of a diagram, illustrating pig's knee cartilage defect model. Fig. 7(A) shows a defect site before injecting a gel-forming composition. Fig. 7(B) shows a gel filled in the defect site. Fig. 7(C) shows the cartilage which was taken out of the PBS and wiped out 5 minutes after the gel-forming composition was injected. Pig's knee cartilage was harvested. A cartilage having a defect site (diameter of 1 cm, about 1 mm in depth) was made by carving out the defect site from the Pig's knee cartilage (Fig. 7(A)). This cartilage is a knee cartilage defect model. Since knee has synovial fluid, PBS of pH 7.4 at 37ºC was supposed to be knee synovial fluid, and the cartilage was put in PBS of pH 7.4 at 37ºC. 4PA-PEG10,000 and 4GS-PEG10,000 were dissolved in 20 mM phosphate buffer solutions of pH 7.4 at a concentration of 10mM, respectively. About 1 mL of each solution was filled in the two-component mixing syringe shown in Fig. 5. The two-component liquid mixture was administered to the cartilage in the PBS solution by pointing the needle tip of the two-component mixing syringe at the vicinity of the cartilage defect site (Fig. 7(B)). The cartilage which was administered the gel was taken out from the PBS solution 5 minutes after the administration, and the gelation of the two-component liquid mixture was confirmed (Fig. 7(C)). It can be seen from the above result that the two-component liquid mixture was hardened in vivo on site. Also, the gel was spread all over the defect site, and high adhesivity of gel to the bone was achieved. Namely, the medical kit of the present invention can administer a gel having pharmaceutical agents to, for example, knee cartilage defect site filled with synovial fluid. This shows that the medical kit of the present invention acts as quite an effective and practical DDS.

### Example 14

### Verification of Chondrogenesis Promoting Effect of TM

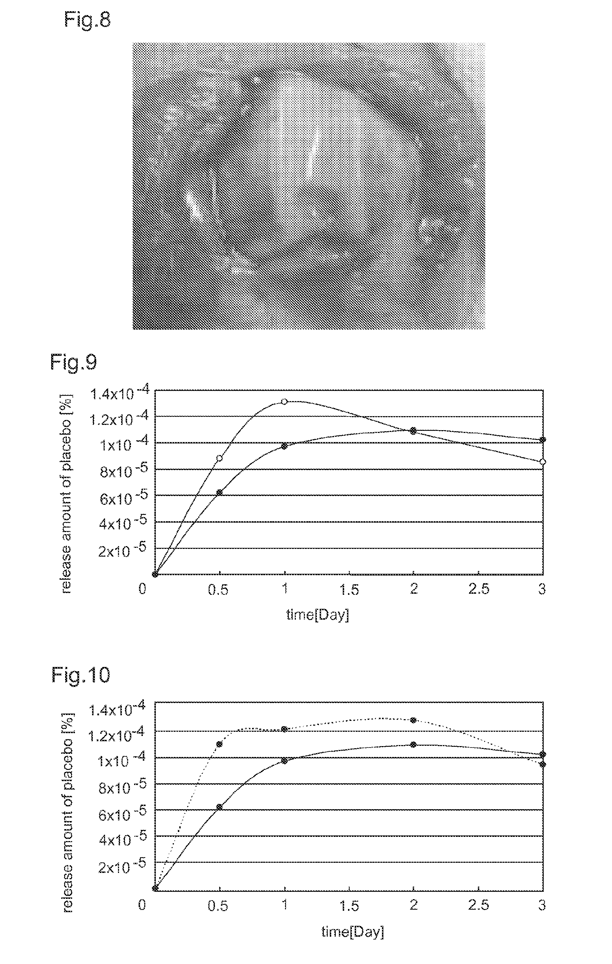

Fig. 8 explains the present example. Fig. 8 is a photograph, in place of a diagram, for verifying the effect of chondrogenesis promotion. Fig. 8 shows rat's knee cartilage defect site. A left knee of a Wistar rat (200g, male) was exposed. The size of the defect site was determined by a trephine of diameter 1.5 mm, and a half layer defect model (1 mm depth) was made by a dental round bur (FIG. 8). 4PA-PEG10,000 and 4GS-PEG10,000 were dissolved in physiologic saline solutions at concentrations of 10mM. TM was added to the first component at a TM concentration in the gel liquid mixture of 1 µM or 10 µM. Also, a control which does not contain TM was prepared. The first component and the second component thus obtained were mixed together in a 0.5 mL sterile tube. The mixture was administered to the cartilage defect site by a sterile spatula before it was gelated. The mixture was gelated for about 3 minutes after the gel containing TM was injected. And then the knee was closed. On the other hand, in the right knee, a half layer defect model was made in the same way, and the control was injected or nothing was injected. Then, chondrogenesis was evaluated histologically two weeks later, four weeks later, six weeks later and eight weeks later. As a result, not specifically shown in figures, it could be seen that cartilage formed gradually.

### Example 15

### Preparation of Drug Release Controlling Carrier "Pseudo-Polyrotaxane"

"Pseudo-Polyrotaxane" was prepared based on methods described in Complex Formation between Poly (ethylene glycol) and α-Cyclodextrin. Akira Harada and Mikiharu Kamachi, Macromolecules 1990, 23, 2821-2823.

α-Cyclodextrin was ("α-100" manufactured by Pearl Ace corporation) was dissolved in a 20mM phosphate buffer solution (pH 7.4) at a concentration of 149 mM. Polyethylene glycol (molecular weight 6,000) was dissolved in this 149 mM α-cyclodextrine solution at a concentration of 1.5%. The resultant 149 mM α-cyclodextrin - 1.5% polyethylene glycol solution was stirred for 10 minutes. After that, the solution was left at room temperature overnight. And then precipitate collected by centrifugation was dried under negative pressure using a desiccator. The resultant dried precipitate was used as a drug release controlling carrier. The pH of a phosphate buffer solution was adjusted to a desired pH level by mixing 200 mM sodium dihydrogen phosphate with 200 mM disodium hydrogen phosphate, and thus prepared phosphate buffer solution of 10 times level was used.

### Drug Release Controlling Carrier Containing Placebos and Preparation for Gel Solution

Methylene Blue and Bromophenol Blue were contained by the drug release controlling carrier as placebos, in place of pharmaceutical agents. Molecular weights of these placebos were respectively 319.85 g/mol and 669.96 g/mol, and these placebos were compounds having benzene rings and/or heterocycles within molecules. So these compounds are considered to have chemical properties relatively similar to those of pharmaceutical agents such as TM. And since these compounds are pigment, they can easily be observed when they are contained in a drug release controlling carrier or when they are released from a drug release controlling carrier.

Methylene Blue as a placebo and the drug release controlling carrier above described were dispersed in 20 mM phosphate buffer solution (pH 7.4) at a concentration of 0.001 % by weight and 20% by weight, respectively. The above fluid dispersion was shaken overnight at 25ºC, and the placebo was contained by the drug release controlling carrier. The resultant fluid dispersion of the drug release controlling carrier containing Methylene Blue was named as gelator solvent No. 1. Also, another fluid dispersion of the drug release controlling carrier was obtained in the same way as the above except that Bromophenol Blue, in place of Methylene Blue, was dispersed at a final concentration of 0.0025%. The resultant fluid dispersion of the drug release controlling carrier containing Bromophenol Blue was named to as gelator solvent No. 2.

### Preparation for Gel Composition Containing Drug Release Carrier Comprising Two Components

Pentaerythritol tetra-polyethylene glycol ether propylamine ("4PA-PEG10,000") (molecular weight 10,000), as the first component, was dissolved in the above gelator solvent No. 1 and gelator solvent No. 2 at a concentration of 10 mM, respectively. The same solvent was used for the first component and the second component. Solution of the 4GS-PEG10,000 which was the second component is unstable in aqueous solvent due to the susceptibility of the active ester to hydrolysis. So it is advisable that the solution is used immediately after preparation. Thus, a desirable preparation method of two fluid components is as follows. The first component is prepared in advance, and the second component is prepared immediately before its use. The medical kit of the present invention is preferred because it can be used effectively for the above method.

4PA-PEG10, 000 and 4GS-PEG10, 000 were dissolved in the gelator solvent No. 1 and the gelator solvent No. 2 respectively at a concentration of 10 mM. Having been uniformly mixed in syringes with a three way stopcock, the mixture was poured into a frame of ϕ 10 mm x 5 mm. Then, the mixture was gelated at room temperature, and the gel thus obtained was used for a confirmatory test of the drug release controlling effect. On the other hand, a control gel was processed from a gelator solvent which was made by adding placebo to the gelator solvent No. 1 and the gelator solvent No. 2 respectively and without adding drug release carrier.

### Repeated Pressurization on the Gel and a Measuring Method of the Placebo Released

The gel thus obtained was placed on the center of a polystyrene container of ϕ 22 mm x 18.7 mm, and 20 mM phosphate buffer solution (pH 7.4) of 3 mL was added to it. Pressurization to the gel soaked in the 20 mM phosphate buffer solution (pH 7.4) was repeated in a certain interval and in a certain rate until gel deformation ratio became 20% using a general physical properties measuring apparatus ("rheometer" manufactured by Sun Science Co., Ltd.) (the pressurized condition: pressurization rate was 1 mm/min and the number of pressurization was once/10 min). The gel soaked phosphate buffer solution was sampled in certain intervals, and the concentration of the placebo released from the gel was measured. On the other hand, a control gel which was prepared at room temperature without being pressurized was also sampled, and the concentration of the placebo released from the control gel was compared to that of the placebo released from the gel.

For the gelator solvent No. 1, absorbance of light of wavelength 615 nm was measured, and a standard curve was made. On the other hand, for the gelator solvent No. 2, absorbance of light of wavelength 595 nm was measured, and a standard curve was made. The concentrations of the placebos released from the gels were calculated based on standard curves.

### Verification of Controlled-Release of the Drug Release Controlling Carrier

Fig. 9 is a graph showing an influence of pressure on the releasing capacity of the drug release controlling carrier of the present invention when the gelator solvent No. 1 was used. The horizontal axis shows time in days, and the vertical axis shows the concentration in percent of Methylene Blue released. The black dots represent the concentrations when the gelator solvent No. 1 was used, and the white dots represent the concentrations when the control was used. The control was prepared in the same way as the gelator solvent No. 1 except that it did not contain the drug release controlling carrier.

As can be seen from the Fig. 9, release amount of the placebo from the control reached a peak level on about day 1, and the release amount decreased largely as time elapsed. On the other hand, when the gelator solvent No. 1 was used, release amount of the placebo remained at approximately the same level after day 1. It can be seen from the results that the drug release controlling carrier of the present invention can achieve quite desirable sustained drug release even when the carrier is suffering from pressure.

### Examination of Change in Sustained Release Property of the Drug Release Controlling Carrier in Accordance with the Presence or Absence of Pressurization

Fig. 10 is a graph showing release amounts of placebo from gels formed from the gelator solvent No. 1 in accordance with the presence or absence of pressure on the gels. Black dots connected by a solid line in Fig. 10 are the same with the black dots shown in Fig. 9. On the other hand, black dots connected by a dotted line in Fig. 10 shows release amount from a gel which contains drug release controlling carrier but was not pressurized.

In Fig. 10, the release amount of placebo from the gel which was not pressurized remains at about the same level from day 0.5 to day 2. But the release amount decreased after day 2.5. So the drug release controlling carrier of the present invention can exert more preferred sustained release property when the carrier suffers from continuous pressure.

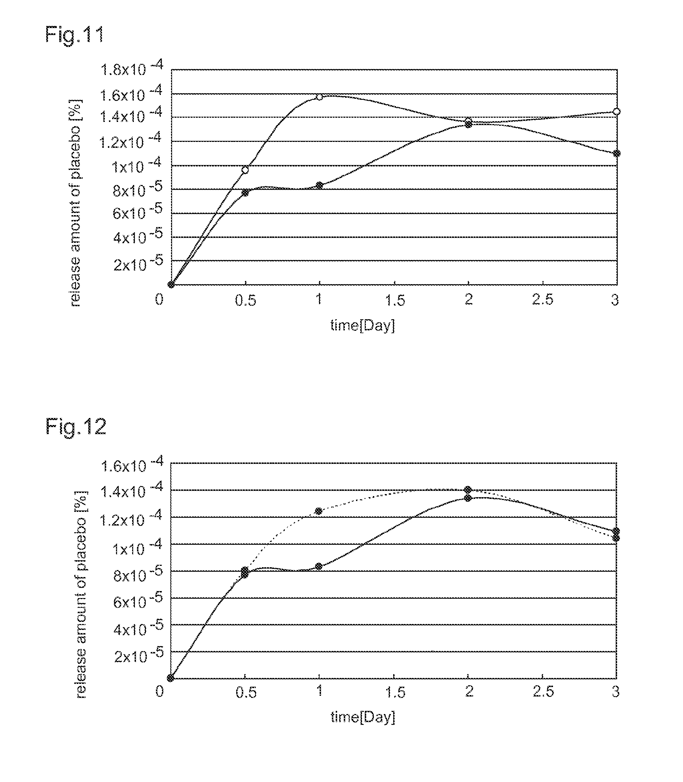

Fig. 11 is a graph showing an influence of pressure on the releasing capacity of the drug release controlling carrier of the present invention when gelator solvent No. 2 was used. When comparing Fig. 9 with Fig. 11, it can be seen that the gel obtained from the gelator solvent No. 1 maintained preferred sustained release property than the gel obtained from the gelator solvent No. 2. These results show that the drug release controlling carriers used in the examples are, in particular, preferably used as carriers of pharmaceutical agents similar to Methylene Blue (e.g., a complex compound with molecular weight of equal to or more than 200 g/mol and equal to or less than 500 g/mol).

Fig. 12 is a graph showing release amounts of placebo from gels formed from the gelator solvent No. 2 in accordance with the presence or absence of pressure on the gels. When the gelator solvent No. 2 was used to form a gel, release amount of placebo from the gel remains constant by applying pressure on the gel, thereby achieving favorable release property.

The gel-forming composition of the present invention is useful as a therapeutic composition for osteoarthritis etc., so it can be used in the field of pharmaceutical industry. Since the administration device of the present invention is useful as a therapeutic device for osteoarthritis etc., it can be effectively used in the field of pharmaceutical industry and device manufacturing industry. Also, the drug release controlling carrier of the present invention can be preferably used in the field of pharmaceutical industry, because it has suitable sustained drug release property.

## Claims

1. A gel-forming composition comprising a first composition and a second composition, the first composition and the second composition becoming gel state when mixed with each other,
wherein the first composition comprises:
a pharmaceutical agent;
a first gelator comprising one or more than one kind of compound represented by the below-described general formula (I) or (II), or a compound including 3 to 8 repeating units represented by the below-described general formula (III); and
a first diluent, and
wherein the second composition comprises:
a second gelator comprising one or more than one kind of compound represented by the below-described general formula (I) or (II), or a compound including 3 to 8 repeating units represented by the below-described general formula (III); and
a second diluent:
X₁ - (OCH₂CH₂)ₙ - X₂ (I)
wherein X₁ and X₂ are the same or different, and each represents - R¹COONHS (where R¹ is a C₁₋₇ alkylene group), -COR¹COONHS, -NOCOR¹-R² (where R² is a maleimide group), -R¹NH₂, -R¹SH, or -CO₂PhNO₂ (where Ph is an o-, m-, or p-phenylene group), and n is an integer of 80 to 1000; 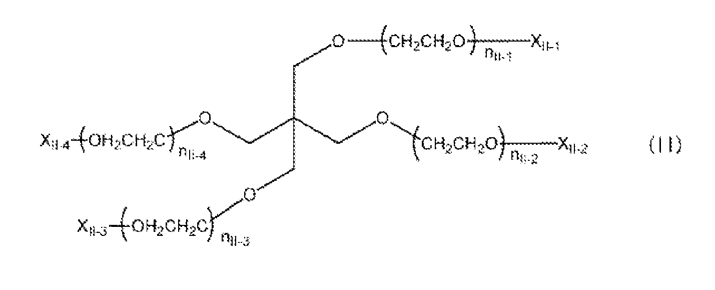 wherein X_{II-1} to X_{II-4} are the same or different and each represents - R¹COONHS (where R¹ is a C₁₋₇ alkylene group), -COR¹COONHS, -NOCOR¹-R² (where R² is a maleimide group), -R¹NH₂, -R¹SH, or -CO₂PhNO₂ (where Ph is an o-, m-, or p-phenylene group), and n_{II-1} to n_{II-4} are the same or different and each represents an integer of 20 to 250; 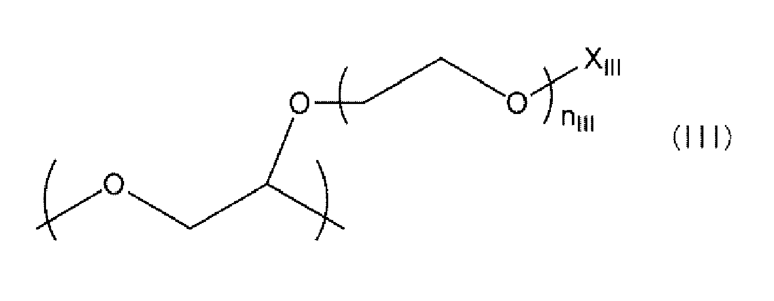 wherein X_{III}, represents -R¹COONHS (where R¹ is a C₁₋₇ alkylene group), - COR¹COONHS, -NOCOR¹-R² (where R² is a maleimide group), -R¹NH₂, -R¹SH, or - CO₂PhNO₂ (where Ph is an o-, m-, or p-phenylene group), and n_{III} represents an integer of 10 to 150.

2. The gel-forming composition as claimed in claim 1,
wherein the first gelator comprises one or more than one kind of compound represented by the general formula (I) or (II),
wherein X₁, X₂ or X_{II-1} to X_{II-4} are the same or different and each represents -NOCOR¹-R² or -R¹NH₂, and
wherein the second gelator comprises one or more than one kind of compound represented by the general formula (I) or (II),
wherein X₁, X₂ or X_{II-1} to X_{II-4} are the same or different and each represents -COR¹COONHS, -R¹SH, or -CO₂PhNO₂ (where Ph is an o-, m-, or p-phenylene group).

3. The gel-forming composition as claimed in claim 1,
wherein the first gelator comprises one or more than one kind of compound represented by the general formula (I) or (II),
wherein X₁, X₂ or X_{II-1} to X_{II-4} are the same or different and each represents -NOCOR¹-R² or -R¹NH₂, and number average molecular weight is 3x10³ to 4x10⁴, and
wherein the second gelator comprises one or more than one kind of compound represented by the general formula (I) or (II),
wherein X₁, X₂ or X_{II-1} to X_{II-4} are the same or different and each represents -COR¹COONHS, -R¹SH, or -CO₂PhNO₂ (where Ph is an o-, m-, or p-phenylene group), and number average molecular weight is 3 x 10³ to 4 x 10⁴.

4. The gel-forming composition as claimed in claim 1,
wherein the diluent is one kind or a mixture of more than one kind of water, phosphate buffer solution, citrate buffer solution, phosphate buffered saline solution or physiologic saline solution.

5. The gel-forming composition as claimed in claim 1,
wherein one or both of the first composition and the second composition further comprises stabilizing agent.

6. The gel-forming composition as claimed in claim 1,
wherein the pharmaceutical agent is an osteogenesis/chondrogenesis promoter, a joint disease therapeutic agent, a preventive and/or therapeutic agent for bone/cartilage disease, a bone-regenerating agent, a bone resorption-suppressing substance, an angiogenesis promoter, or an anticancer agent.

7. The gel-forming composition as claimed in claim 1,
wherein the pharmaceutical agent comprises thieno indazole derivative represented by below-described general formula (IV), prostaglandin A1 derivative, vitamin D derivative, vitamin K₂ derivative, eicosapentaenoic acid, benzyl sulfonic acid, bisphosphonic acid derivative, sex hormone derivative, phenol sulfo phthalein derivative, benzothiopyran or benzothiepine derivative, menatetrenone derivative, helioxanthine derivative, dihydrobenz [c] thiophene compound, tricyclic thiophene compound, Indian hedgehog (Ihh) or agonist of the signaling pathway thereof, PTHrP (PTH-related peptide) or agonist of the signaling pathway thereof, pharmaceutically acceptable salt thereof, or pharmaceutically acceptable solvate thereof: 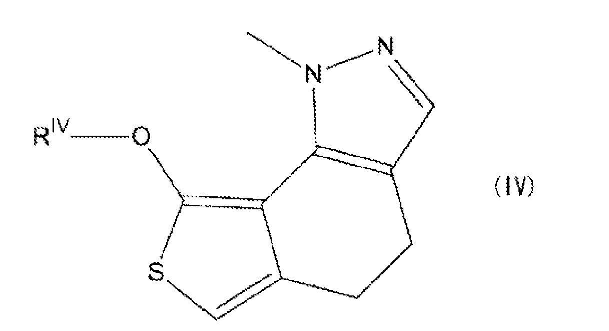 wherein R^{1V} represents a carboxyamido group.

8. The gel-forming composition as claimed in claim 1,
wherein the pharmaceutical agent comprises trafermin, pharmaceutically acceptable salt thereof, pharmaceutically acceptable solvate thereof or prodrug thereof.

9. The gel-forming composition as claimed in claim 1,
wherein the pharmaceutical agent comprises a gene.

10. The gel-forming composition as claimed in claim 1, further comprising controlled-release formulation for controlling release of the pharmaceutical agent after gel formation is completed.

11. The gel-forming composition as claimed in claim 1,
wherein the pharmaceutical agent is a ligand.

12. The gel-forming composition as claimed in claim 1, used as a wound coating material.

13. The gel-forming composition as claimed in claim 1, used as a wound coating material,
wherein the pharmaceutical agent comprises trafermin, a pharmaceutically acceptable salt thereof, a pharmaceutically acceptable solvate thereof or a prodrug thereof.

14. The gel-forming composition as claimed in claim 1, further comprising polyrotaxane or pseudo-polyrotaxane as a drug release controlling carrier.

15. The gel-forming composition as claimed in claim 1, further comprising polyrotaxane or pseudo-polyrotaxane as a drug release controlling carrier,
wherein the polyrotaxane or the pseudo-polyrotaxane comprises:
one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and
one or more than one kind of chain molecules selected from a group consisting of "polyalkylene glycol, copolymer of polyalkylene glycol, polyether, polyolefin, polyorganosiloxane, derivative thereof, pharmaceutically acceptable salts thereof, and pharmaceutically acceptable solvate thereof".

16. The gel-forming composition as claimed in claim 1, further comprising pseudo-polyrotaxane as drug release controlling carrier,
wherein the pseudo-polyrotaxane comprises:
cyclodextrin; and
polyethylene glycol, polypropylene glycol, or copolymer of polyethylene glycol and polypropylene glycol.

17. The gel-forming composition as claimed in claim 1, the gel-forming composition being used for medical treatment of bone/cartilage disease, the gel-forming composition further comprising polyrotaxane or pseudo-polyrotaxane as drug release controlling carrier,
wherein the polyrotaxane or the pseudo-polyrotaxane comprises:
one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and
one or more than one kind of chain molecules selected from a group consisting of "polyalkylene glycol, copolymer of polyalkylene glycol, polyether, polyolefin, polyorganosiloxane, derivative thereof, pharmaceutically acceptable salt thereof, and pharmaceutically acceptable solvate thereof".

18. The gel-forming composition as claimed in claim 1, the gel-forming composition being used for medical treatment of knee joint disease, the gel-forming composition further comprising polyrotaxane or pseudo-polyrotaxane as drug release controlling carrier,
wherein the polyrotaxane or the pseudo-polyrotaxane comprises:
one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivative thereof, pharmaceutically acceptable salt thereof, and pharmaceutically acceptable solvate thereof", and
one or more than one kind of chain molecules selected from a group consisting of "polyalkylene glycol, copolymer of polyalkylene glycol, polyether, polyolefin, polyorganosiloxane, derivative thereof, pharmaceutically acceptable salt thereof, and pharmaceutically acceptable solvate thereof".

19. The gel-forming composition as claimed in claim 1, the gel-forming composition being used for medical treatment of knee joint disease, the gel-forming composition further comprising pseudo-polyrotaxane as drug release controlling carrier,
wherein the pseudo-polyrotaxane comprises:
cyclodextrin; and
polyethylene glycol, polypropylene glycol, or copolymer of polyethylene glycol and polypropylene glycol.

20. The gel-forming composition as claimed in claim 1, the gel-forming composition being used for medical treatment of knee joint disease, the gel-forming composition further comprising pseudo-polyrotaxane as drug release controlling carrier,
wherein the pseudo-polyrotaxane comprises:
cyclodextrin; and
polyethylene glycol, polypropylene glycol, or copolymer of polyethylene glycol and polypropylene glycol, and
wherein the pharmaceutical agent comprises thieno indazoles derivative represented by the above-described general formula (IV), prostaglandin A1 derivative, vitamin D derivative, vitamin K₂ derivative, eicosapentaenoic acid, benzyl sulfonic acid, bisphosphonic acid derivative, sex hormone derivative, phenol sulfo phthalein derivative, benzothiopyran or benzo thiepine derivative, menatetrenone derivative, helioxanthine derivative, dihydrobenz [c] thiophene compound, or tricyclic thiophene compound, Indian hedgehog (Ihh) or agonist of the signaling pathway thereof, PTHrP (PTH-related peptide) or agonist of the signaling pathway thereof, pharmaceutically acceptable salt thereof, or pharmaceutically acceptable solvate thereof.

21. An administration device (1) containing the gel-forming composition as claimed in claim 1, the administration device (1) comprising:
two containing rooms (2, 3) containing the first composition and the second composition, the two containing rooms (2, 3) spatially isolating the compositions from each other;
moving parts (4a, 4b) being connected to the two containing rooms (2, 3), the moving parts (4a, 4b), wherein the first composition and the second composition can move within the moving parts (4a, 4b) respectively;
a mixing part (5) mixing the first composition and the second composition, the first composition and the second composition having moved through the moving parts (4a, 4b); and
a discharge part (6) discharging the first composition and the second composition, the first composition and the second composition having been mixed in the mixing part (5).

22. The administration device (1) as claimed in claim 21,
wherein the discharge part (6) has a spray port.

23. An administration device (1) containing the gel-forming composition as claimed in claim 1, the administration device (1) comprising:
two containing rooms (2, 3) containing the first composition and the second composition, the two containing rooms (2, 3) spatially isolating the compositions from each other;
moving parts (4a, 4b) being connected to the two containing rooms (2, 3), the moving parts (4a, 4b), wherein the first composition and the second composition can move within the moving parts (4a, 4b) respectively;
discharge parts (6a, 6b) discharging the first composition and the second composition separately, the first composition and the second composition having moved through the moving parts (4a, 4b).

24. The administration device (1) as claimed in claim 23,
wherein the discharge parts (6a, 6b) have spray ports.

25. A medical kit comprising:
a pharmaceutical agent;
a first gelator comprising one or more than one kind of a compound represented by the general formula (I) or (II), or a compound including 3 to 8 repeating units represented by the general formula (III);
a second gelator comprising one or more than one kind of a compound represented by the general formula (I) or (II), or a compound including 3 to 8 repeating units represented by the general formula (III);
a diluent; and
an administration device comprising a two pack type syringe,
wherein the administration device comprises:
two containing rooms (2, 3) containing a first composition and a second composition, the first composition including the pharmaceutical agent, the first gelator, and the diluent, the second composition including the second gelator and the diluent, the two containing rooms (2, 3) spatially isolating the compositions from each other;
moving parts (4a, 4b) being connected to the two containing rooms (2, 3), the moving parts (4a, 4b), wherein the first composition and the second composition can move within the moving parts (4a, 4b) respectively;
a mixing part (5) mixing the first composition and the second composition, the first composition and the second composition having moved through the moving parts (4a, 4b); and
a discharge part (6) discharging the first composition and the second composition, the first composition and the second composition having been mixed in the mixing part (5), or
wherein the administration device comprises:
two containing rooms (2, 3) containing the first composition and the second composition, the two containing rooms (2, 3) spatially isolating the compositions from each other;
moving parts (4a, 4b) being connected to the two containing rooms (2, 3), the moving parts (4a, 4b), wherein the first composition and the second composition can move within the moving parts (4a, 4b)respectively; and
discharge parts (6a, 6b) discharging the first composition and the second composition separately, the first composition and the second composition having moved through the moving parts (4a, 4b).

26. The medical kit as claimed in claim 25,
wherein the discharge part/parts (6 or 6a, 6b) have spray port/ports.

27. The medical kit as claimed in claim 26, further comprising stabilizing agent.

28. A drug release controlling carrier for controlling drug release, the drug release controlling carrier comprising polyrotaxane or pseudo-polyrotaxane,
wherein the polyrotaxane or the pseudo-polyrotaxane comprises:
one or more than one kind of cyclic molecules selected from a group consisting of "cyclodextrin, crown ether, benzo crown, dibenzo crown, dicyclohexano crown, cyclophane, calixarene, cucurbituril, bipyridinium salt, derivatives thereof, pharmaceutically acceptable salt thereof, and pharmaceutically acceptable solvates thereof", and
one or more than one kind of chain molecules selected from a group consisting of "polyalkylene glycol, copolymer of polyalkylene glycol, polyether, polyolefin, polyorganosiloxane, derivative thereof, pharmaceutically acceptable salts thereof, and pharmaceutically acceptable solvate thereof".

29. The drug release controlling carrier as claimed in claim 28 for controlling drug release, the drug release controlling carrier comprising pseudo-polyrotaxane,
wherein the pseudo-polyrotaxane comprises:
cyclodextrin as the cyclic molecule; and
polyethylene glycol, polypropylene glycol, or copolymer of polyethylene glycol and polypropylene glycol as the chain molecule.

30. The drug release controlling carrier as claimed in claim 28 for sustained drug release by applying pressure, the drug release controlling carrier comprising pseudo-polyrotaxane,
wherein the pseudo-polyrotaxane comprises:
cyclodextrin as the cyclic molecule; and
polyethylene glycol, polypropylene glycol, or copolymer of polyethylene glycol and polypropylene glycol as the chain molecule.

31. The drug release controlling carrier as claimed in claim 28 being used for treatment of knee joint, the drug release controlling carrier comprising pseudo-polyrotaxane,
wherein the pseudo-polyrotaxane comprises:
cyclodextrin as the cyclic molecule; and
polyethylene glycol, polypropylene glycol, or copolymer of polyethylene glycol and polypropylene glycol as the chain molecule.
